# EUROPEAN PATENT APPLICATION

(11) **EP 4 841 269 A1**
(43) Date of publication of application: **18.11.2026**
(21) Application number: 26175775.1
(22) Date of filing: 30.04.2026
(51) Int. Cl.: G01N 35/10, G01N 21/25, G01N 33/49, G01N 1/38, G01N 21/82

(54) **SAMPLE ANALYZER AND SAMPLE ANALYSIS METHOD FOR PLATELET AGGREGATION ABILITY OF A BLOOD SAMPLE**

(30) Priority: 12.05.2025 JP 2025079528
(71) Applicant: SYSMEX CORPORATION, Kobe-shi Hyogo 651-0073 (JP)
(72) Inventor: SAKAYORI, Tasuku, Kobe-shi, Hyogo, 651-0073 (JP); KAWANAMI, Yoji, Kobe-shi, Hyogo, 651-0073 (JP)
(74) Representative: Becker, Eberhard

(57) **Abstract**

Disclosed is a sample analyzer configured to analyze a platelet aggregation ability of a blood sample, being capable of performing: a first measurement operation of preparing a measurement specimen in a first reaction container by dispensing a platelet-rich plasma sample and an inducing reagent for inducing platelet aggregation, and optically interrogating the measurement specimen in which the platelet aggregation is induced in the presence of the inducing reagent, and a second measurement operation of preparing a reference specimen in a second reaction container by dispensing the platelet-rich plasma sample without the inducing reagent, and optically interrogating the reference specimen in which a spontaneous platelet aggregation can be observed in the absence of the induced platelet aggregation.

## Description

### BACKGROUND

The present invention relates to a sample analyzer and a sample analysis method, and more particularly to a sample analyzer and a sample analysis method for measuring a platelet aggregation ability of a blood sample.

Conventionally, tests for platelet aggregation ability have been performed using a blood coagulation test apparatus. As disclosed in International Society on Thrombosis and Haemostasis (ISTH) guidelines "Recommendations for the standardization of light transmission aggregometry: a consensus of the working party from the platelet physiology subcommittee of SSC/ISTH" Journal of Thrombosis and Haemostasis, 11: 1183-1189, 2013, for measurement of platelet aggregation ability, a platelet-rich plasma (PRP) sample containing many platelets and a platelet-poor plasma (PPP) sample substantially not containing platelets, which are obtained by performing two types of centrifugation on a whole blood sample collected from a patient, are used. The platelet aggregation ability of a sample is calculated based on optical information obtained by measuring a platelet-poor plasma sample and optical information obtained by measuring a platelet-rich plasma sample to which a reagent containing an inducing agent for inducing platelet aggregation is added.

### SUMMARY

In some platelet-rich plasma samples, spontaneous aggregation occurs in which platelets aggregate without adding an inducing agent due to tissue factors that may be mixed during blood collection. Further, it has been reported that spontaneous platelet aggregation may also occur in samples collected from patients having diseases such as cerebral infarction, myocardial infarction, and von Willebrand disease. Thus, for a sample in which spontaneous platelet aggregation is suspected, the platelet aggregation ability of the sample may not be correctly evaluated.

The disclosure provides a sample analyzer (1) configured to analyze a platelet aggregation ability of a blood sample, comprising: a measurement unit (2) comprising: a measurement specimen preparator (12) configured to prepare a measurement specimen in a reaction container (108), the measurement specimen preparator (12) comprising: (a1) a sample dispenser (120a, 120b, 150a, 150b) configured to dispense a plasma sample prepared from the blood sample into the reaction container (108), and (a2) a reagent dispenser (120a, 120b, 150a, 150b) configured to dispense a reagent into the reaction container (108); and a detector (13) configured to optically interrogate the measurement specimen, the detector (13) comprising: (b1) a light source (132) configured to irradiate the measurement specimen in the reaction container (108) with light, and (b2) a light receiver (133) configured to sense transmitted light or scattered light from the measurement specimen irradiated with light, wherein the detector (13) is configured to obtain optical information corresponding to an intensity of the sensed transmitted or scattered light, wherein the measurement unit (2) is capable of performing: a first measurement operation of preparing the measurement specimen in a first reaction container (108) by dispensing a platelet-rich plasma sample as the plasma sample and an inducing reagent for inducing platelet aggregation as the reagent, and optically interrogating the measurement specimen in which the platelet aggregation is induced in the presence of the inducing reagent, and a second measurement operation of preparing a reference specimen in a second reaction container (108) by dispensing the platelet-rich plasma sample without the inducing reagent, and optically interrogating the reference specimen in which a spontaneous platelet aggregation can be observed in the absence of the induced platelet aggregation; and a controller (210, 213) configured to control the measurement unit (2), wherein the controller (210, 213) is configured to execute a first analysis of the platelet aggregation ability of the blood sample based on the optical information obtained by the first measurement operation, and the controller (210, 213) is further configured to execute a second analysis of a spontaneous platelet aggregation of the blood sample based on the optical information obtained by the second measurement operation.

The disclosure provides a sample analysis method for measuring a platelet aggregation ability of a blood sample, comprising: preparing, by a measurement specimen preparator (12) of a measurement unit (2), a reference specimen in a reaction container (108), the reference specimen containing a platelet-rich plasma sample prepared from the blood sample and not containing an inducing reagent for inducing platelet aggregation, wherein the preparing the reference specimen includes dispensing the platelet-rich plasma sample without the inducing reagent into the reaction container (108); optically interrogating, by a detector (13) of the measurement unit (2), the reference specimen by irradiating the reference specimen in the reaction container with light and sensing transmitted light or scattered light from the reference specimen irradiated with light, whereby obtaining optical information corresponding to an intensity of the sensed transmitted or scattered light from the reference specimen; executing an analysis of a spontaneous platelet aggregation of the blood sample based on the optical information obtained by optically interrogating the reference specimen; preparing, by the measurement specimen preparator (12), a measurement specimen in another reaction container (108), the measurement specimen containing the platelet-rich plasma sample and the inducing reagent, wherein the preparing the measurement specimen includes dispensing the platelet-rich plasma sample and the inducing reagent into said another reaction container (108); optically interrogating, by the detector (13), the measurement specimen by irradiating the measurement specimen in said another reaction container (108) with light and sensing transmitted light or scattered light from the measurement specimen irradiated with light, whereby obtaining optical information corresponding to an intensity of the sensed transmitted or scattered light from the measurement specimen; and
executing an analysis of the platelet aggregation ability of the blood sample based on the optical information obtained by optically interrogating the measurement specimen.

According to the sample analyzer and the sample analysis method of the present disclosure, information regarding spontaneous platelet aggregation of a blood sample can be easily known.

### BRIEF DESCRIPTION OF THE DRAWINGS

FIG. 1 is a view showing an appearance of a sample analyzer which is an example of an embodiment.
FIG. 2 is a plan view showing a configuration of the sample analyzer.
FIG. 3 is a block diagram showing a configuration of the sample analyzer.
FIG. 4 is a view showing an example of a main menu screen.
FIG. 5 is a view showing an example of an order screen.
FIG. 6 is a flowchart showing an example of a sample analysis method.
FIG. 7 is a flowchart showing an example of a third measurement operation.
FIG. 8 is a flowchart showing an example of a second measurement operation.
FIG. 9 is a flowchart showing an example of a first measurement operation.
FIG. 10 is a view showing an example of a setting screen regarding sample measurement.
FIG. 11 is a view showing an example of a setting screen regarding measurement of spontaneous platelet aggregation.
   (a) and (b) of FIG. 12 are views showing an example of a measurement result of spontaneous platelet aggregation.
FIG. 13 is a view showing an example of a display screen of measurement results.
FIG. 14 is a flowchart showing another example of the sample analysis method.
FIG. 15 is a flowchart showing another example of the sample analysis method.
FIG. 16 is a flowchart showing another example of the sample analysis method.
FIG. 17 is a flowchart showing another example of the sample analysis method.
FIG. 18 is a flowchart showing another example of the second measurement operation.

### DESCRIPTION OF THE EMBODIMENTS

Hereinafter, embodiments of a sample analyzer and a sample analysis method according to the present invention will be described in detail with reference to the drawings. The embodiments described below are merely examples, and the present invention is not limited to the following embodiments. In addition, the present invention includes forms obtained by selectively combining each component of a plurality of embodiments and modifications described below.

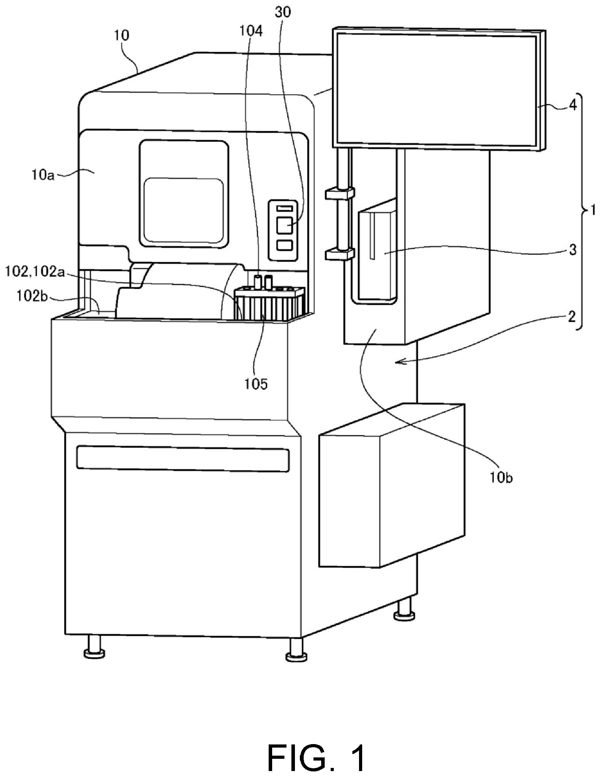

FIG. 1 is a perspective view showing an appearance of a sample analyzer 1 which is an example of an embodiment. As shown in FIG. 1, the sample analyzer 1 includes a housing 10 having a substantially rectangular parallelepiped outer shape. The sample analyzer 1 is a blood coagulation measurement apparatus, and is an apparatus capable of measuring a platelet aggregation ability of a blood sample in addition to measuring a coagulation function of the blood sample. The platelet aggregation ability is a parameter indicating the ease of platelet aggregation. The housing 10 has a front cover 10a that can be opened and closed. The front cover 10a slides or rotates up and down, and the inside of the apparatus can be opened by opening the front cover 10a. Further, the sample analyzer 1 includes a transport unit 102 for transporting a sample, and a measurement start button 30.

The transport unit 102 is provided at a central portion in a height direction at the front of the sample analyzer 1. The transport unit 102 includes a rack placement part 102a, which is a place where a sample rack 105 is placed, and a rack collection part 102b, which is a place where the sample rack 105 after measurement is carried out from the inside of the housing 10. The sample rack 105 holds a plurality of sample containers 104 containing samples. As will be described in detail later, when a measurement operation of a sample is started, the sample rack 105 placed on the rack placement part 102a is transported into the housing 10, and after the measurement of the sample is completed, is carried out from the inside of the housing 10 to the rack collection part 102b.

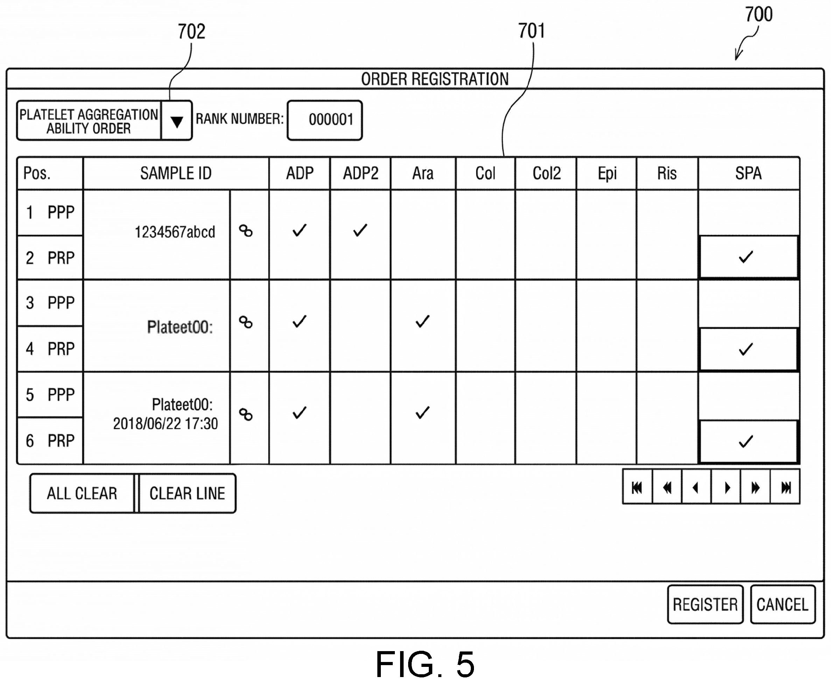

The measurement start button 30 is provided above the rack placement part 102a at the front of the sample analyzer 1. When the measurement start button 30 is operated after a sample rack 105 holding sample containers 104 is placed on the rack placement part 102a by an operator, the sample rack 105 is transported into the housing 10 and measurement of the samples is started. Note that in a case where sample information such as a sample identification number (sample ID) and measurement items is manually input, the measurement start button 30 is operated after the sample information is input on the order screen 700 (see FIG. 5 described later).

The sample analyzer 1 includes a measurement unit 2, a control unit 3, and a display 4. The measurement unit 2 is a unit where preparation of a measurement specimen and obtaining of optical information are performed. The control unit 3 is a computer that analyzes the optical information obtained by the measurement unit 2, and is connected so as to be communicable with the measurement unit 2. In the example shown in FIG. 1, the display 4 is attached to a side surface of the housing 10, and the control unit 3 is accommodated in a side rack 10b attached behind the display 4.

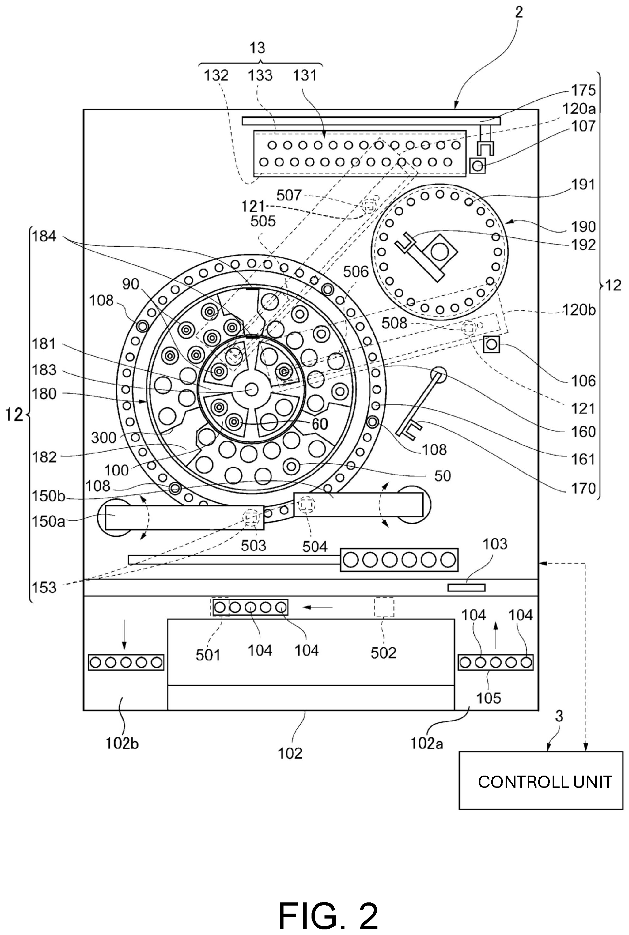

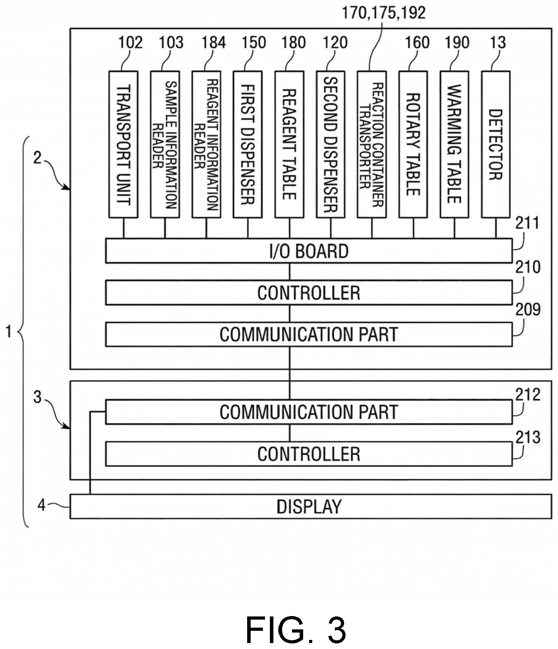

A controller 213 (see FIG. 3) of the control unit 3 has a function of obtaining an analysis result regarding a platelet aggregation ability of a blood sample based on optical information obtained by a detector 13 (see FIG. 2) of the measurement unit 2, and may be connected so as to be communicable with a host computer in which sample information such as a sample ID and measurement items is registered. The display 4 is an example of a display unit that displays an analysis result regarding the platelet aggregation ability and the like. Further, the display 4 is a touch-panel type display that also functions as an input unit. The display 4 is connected to the control unit 3 and is connected so as to be communicable with the measurement unit 2 via the control unit 3. For example, an operation signal of the display 4, which is a touch panel, is transmitted to the measurement unit 2 via the control unit 3. Note that the function of the control unit 3 may be built into the measurement unit 2, or the display 4 may be integrated with the measurement unit 2.

The sample analyzer 1 is an apparatus that measures blood coagulation function, platelet aggregation ability, and the like by, for example, a clotting method, a chromogenic method, an immunoassay method, and an aggregation method. Hereinafter, the configuration and method regarding the measurement of platelet aggregation ability will be described in detail.

FIG. 2 is a plan view showing an internal structure of the sample analyzer 1. As shown in FIG. 2, the sample analyzer 1 includes a measurement unit 2 having: a measurement specimen preparator 12 configured to be capable of preparing a measurement specimen in a reaction container 108 by dispensing a plasma sample prepared from a blood sample and a reagent into the reaction container 108; and a detector 13 configured to irradiate the measurement specimen in the reaction container 108 with light, sense transmitted light or scattered light (transmitted light in the present embodiment) from the measurement specimen irradiated with the light, and obtain optical information corresponding to an intensity of the sensed light. The measurement specimen preparator 12 prepares the measurement specimen in the reaction container 108 by dispensing the sample contained in the sample container 104 and reagents contained in reagent containers 50, 60, and 90 into the reaction container 108 using dispensers 120a, 120b, 150a, and 150b. The detector 13 irradiates the measurement specimen in the reaction container 108 with light, senses transmitted light or scattered light (transmitted light in the present embodiment) from the sample irradiated with the light, and obtains optical information corresponding to the intensity of the sensed light.

The sample used in the measurement of the platelet aggregation ability is a plasma sample prepared from a blood sample (whole blood sample). The plasma samples are, for example, a platelet-rich plasma (PRP) sample and a platelet-poor plasma (PPP) sample. The PRP sample is a sample in which platelets are concentrated from a blood sample. The PPP sample is a sample prepared from a blood sample or a PRP sample and substantially does not contain platelets.

The PRP sample and the PPP sample are prepared by performing two types of centrifugal treatments on a whole blood sample collected from a subject. The PRP sample is, for example, a supernatant obtained by centrifuging a whole blood sample to which an anticoagulant such as sodium citrate has been added at 200 × g for 10 minutes. Note that × g is a unit of centrifugal force. The PPP sample is, for example, a supernatant obtained by centrifuging a whole blood sample to which an anticoagulant has been added at 200 × g for 10 minutes and further at 1500 × g for 15 minutes. The sample container 104 containing the PRP sample and the sample container 104 containing the PPP sample are arranged as a pair in the same sample rack 105.

An inducing reagent for inducing platelet aggregation includes a substance (inducing agent) for inducing platelet aggregation. Examples of the inducing agent include adenosine diphosphate (ADP), collagen, epinephrine, arachidonic acid, ristocetin, and protease-activated receptor 1-activating peptide (PAR1-AP). For ADP, REVOHEM ADP (registered trademark) can be used. For collagen, REVOHEM Collagen (registered trademark) can be used. For epinephrine, REVOHEM Epinephrine (registered trademark) can be used. For arachidonic acid, REVOHEM Arachidonic Acid (registered trademark) can be used. For ristocetin, REVOHEM Ristocetin (registered trademark) can be used.

The sample analyzer 1 is configured to be capable of evaluating the platelet aggregation ability of a blood sample by preparing a first measurement specimen containing a PRP sample and an inducing reagent and obtaining first optical information. The sample analyzer 1 is further configured to be capable of evaluating a spontaneous platelet aggregation of a blood sample by preparing a second measurement specimen containing the PRP sample and not containing the inducing reagent and obtaining second optical information. According to the sample analyzer 1, spontaneous aggregation information regarding the spontaneous platelet aggregation of a blood sample can be easily known. Spontaneous platelet aggregation is aggregation of platelets that occurs in an environment where no inducing reagent exists. The spontaneous aggregation information is information indicating ease of spontaneous platelet aggregation of a blood sample. For a sample in which spontaneous platelet aggregation occurs, the platelet aggregation ability of the sample may not be correctly evaluated. Therefore, if the spontaneous aggregation information such as presence or absence of spontaneous platelet aggregation or a level of spontaneous aggregation can be easily confirmed, usability is improved and reliability of measurement of the platelet aggregation ability is also improved. In the present specification, in contrast to spontaneous platelet aggregation, platelet aggregation in the first measurement specimen prepared by mixing the PRP sample and the inducing reagent may be referred to as "normal aggregation."

The measurement unit 2 is configured to be capable of performing: a first measurement operation of preparing a first measurement specimen containing a PRP sample and an inducing reagent and obtaining first optical information; and a second measurement operation of preparing a second measurement specimen containing the PRP sample and not containing the inducing reagent and obtaining second optical information. The measurement unit 2 is further configured to be capable of performing a third measurement operation of preparing a third measurement specimen containing a PPP sample and measuring third optical information. The measurement specimen preparator 12 prepares the first measurement specimen by dispensing the inducing reagent into the reaction container 108 containing the PRP sample, and prepares the third measurement specimen by dispensing a diluent into the reaction container 108 containing the PPP sample. The second measurement specimen may be the PRP sample itself, or may be prepared by dispensing a diluent into the PRP sample.

The controller 213 of the control unit 3 obtains an analysis result regarding the platelet aggregation ability of a blood sample based on the optical information obtained by the measurement unit 2. The controller 213 displays the analysis result regarding the platelet aggregation ability of the blood sample on the display 4. When the measurement unit 2 performs the first measurement operation and the second measurement operation, the controller 213 obtains a measurement value of the platelet aggregation ability of the blood sample based on the first optical information obtained by the first measurement operation, and obtains spontaneous aggregation information regarding a spontaneous platelet aggregation of the blood sample based on the second optical information obtained by the second measurement operation.

As will be described in detail later, when the measurement unit 2 performs the first measurement operation, the second measurement operation, and the third measurement operation, the controller 213 of the control unit 3 obtains a measurement value of the platelet aggregation ability of the sample based on the first optical information obtained by the first measurement operation and the third optical information obtained by the third measurement operation. Further, the controller 213 of the control unit 3 obtains spontaneous aggregation information regarding a spontaneous platelet aggregation of the sample based on the second optical information obtained by the second measurement operation and the third optical information obtained by the third measurement operation. That is, the third optical information is used for obtaining both the measurement value of the platelet aggregation ability and the spontaneous aggregation information.

The measurement specimen preparator 12 comprises a reagent table 180. The reagent table 180 is a circular table. In the reagent table 180, a plurality of container racks 100 and 300 are arranged along a circumferential direction of the table. Reagent containers 50, 60, and 90 containing reagents are arranged in the container racks 100 and 300. The arrangement of the reagent containers 50, 60, and 90 in the container racks 100 and 300 and the arrangement of the container racks 100 and 300 on the reagent table 180 are performed in a state where an operator opens the front cover 10a to open the inside of the apparatus. The reagent containers 50, 60, and 90 are appropriately selected according to measurement items. Hereinafter, an example in which a diluent is contained in the reagent container 50 and an inducing reagent is contained in the reagent container 60 will be described.

The reagent table 180 includes a first table 181 having a circular shape in plan view and a second table 182 having an annular shape in plan view provided on an outer periphery of the first table 181. In the example shown in FIG. 2, four container racks 100 are arranged along the circumferential direction on the first table 181. Three large container racks 300 are arranged along the circumferential direction on the second table 182. The first table 181 and the second table 182 are independently rotatable in the circumferential direction about a rotation axis 183 by a rotation mechanism including an electric motor, and the reagent containers 50, 60, and 90 can be respectively moved to predetermined positions by rotation.

The measurement specimen preparator 12 includes a reagent information reader 184 that reads information attached to each of the reagent containers 50, 60, and 90 and the container racks 100 and 300. For example, this information is attached to each container and rack with a barcode, and the reagent information reader 184 includes a barcode reader. The first table 181 and the second table 182 can move the container racks 100 and 300 and each container held in the racks to a reading position facing the reagent information reader 184. By reading the information with the reagent information reader 184, respective positions of the reagent containers 50, 60, and 90 on the reagent table 180 can be identified.

The information attached to the reagent containers 50, 60, and 90 includes, for example, identification numbers (IDs) of the reagent containers 50, 60, and 90, names of reagents, types of reagents, concentrations of reagents, lot numbers, expiration dates, and the like.

The measurement specimen preparator 12 has dispensers 150a and 150b. Each of the dispensers 150a and 150b has a dispensing arm that pivotally holds a aspiration pipette 153 for dispensing. The aspiration pipette 153 is connected to a pump and configured to be capable of sucking and dispensing a predetermined amount of liquid.

As described above, the sample analyzer 1 includes the transport unit 102 including the rack placement part 102a and the rack collection part 102b. The transport unit 102 transports the sample rack 105 placed on the rack placement part 102a by an operator into the housing 10, and places the sample containers 104 held in the sample rack 105 at sample aspiration positions 501 and 502 of the measurement unit 2. Further, the transport unit 102 has a sample information reader 103 arranged in a transport path of the sample rack 105. The sample information reader 103 includes, for example, a barcode reader.

To the sample container 104, a label recording sample information in a barcode or the like is attached. The sample information includes, for example, information such as a sample ID, a type of sample, a provider of the sample, and measurement items. As the information regarding the type of sample, information as to whether the sample is a PRP sample or a PPP sample is included. In a case where the sample information is registered in a host computer, the operator does not need to input the sample information on the order screen 700, and may place the sample rack 105 on the rack placement part 102a of the transport unit 102 and operate the measurement start button 30. In this case, the sample information reader 103 reads the barcode of the sample container 104 held in the sample rack 105, inquires of the host computer for information necessary for measurement, and automatically registers a measurement order.

The measurement specimen preparator 12 comprises a rotary table 160 that transports the reaction container 108. The rotary table 160 is arranged outside the reagent table 180. The rotary table 160 has a ring shape in plan view and is rotatable in the circumferential direction. The rotary table 160 is provided with a plurality of holding holes 161 arranged along the circumferential direction. One reaction container 108 can be arranged in each holding hole 161. Into the reaction container 108, for example, a PRP sample and an inducing reagent are dispensed, and a measurement specimen is prepared in the reaction container 108. The reaction container 108 is, for example, a cuvette. In the reaction container 108 used for the measurement of the platelet aggregation ability, a stirring bar is placed.

Samples are dispensed into the reaction containers 108 by the dispensers 150a and 150b. The dispenser 150a moves the aspiration pipette 153 to suck a predetermined amount of sample from the sample container 104 arranged at the sample aspiration position 501 of the transport unit 102. The dispenser 150b moves the aspiration pipette 153 to suck a predetermined amount of sample from the sample container 104 arranged at the sample aspiration position 502 of the transport unit 102. The dispensers 150a and 150b having sucked the samples dispense the samples into the reaction containers 108 arranged at sample dispensing positions 503 and 504 of the rotary table 160.

The measurement specimen preparator 12 includes a reaction container transporter 170 capable of transporting the reaction container 108, and a warming table 190 that holds and warms the reaction container 108. The reaction container transporter 170 takes out the reaction container 108 from the holding hole 161 of the rotary table 160 by gripping the reaction container 108, transfers the reaction container 108 to the warming table 190, and places it on the warming table 190. Further, the reaction container transporter 170 places the reaction container 108 at a reagent dispensing position 508. The warming table 190 is an example of a warmer that warms the reaction container 108 transferred by the reaction container transporter 170 to a predetermined temperature.

The warming table 190 is a circular table having a heater built therein, and includes a plurality of holding holes 191 for holding a plurality of reaction containers 108, respectively. A plurality of holding holes 191 are arranged along the circumferential direction of the warming table 190. The warming table 190 is rotatable in the circumferential direction, and while warming the reaction containers 108 to a predetermined temperature by the heater, transfers the reaction containers 108 arranged in the plurality of holding holes 191 in the circumferential direction by rotation. The reaction container transporter 192 takes out the reaction container 108 from the holding hole 191 and places the reaction container 108 at reagent dispensing positions 507 and 508.

The measurement specimen preparator 12 comprises dispensers 120a and 120b. Each of the dispensers 120a and 120b comprises a aspiration pipette 121 for dispensing. The dispensers 120a and 120b move the aspiration pipettes 121 above the reagent containers 50, 60, and 90 arranged at predetermined reagent aspiration positions 505 and 506 on the reagent table 180, and aspirate a predetermined amount of reagent from the reagent containers 50, 60, and 90. Thereafter, the dispensers 120a and 120b move above the reaction containers 108 arranged at the reagent dispensing positions 507 and 508, respectively, and dispense a predetermined amount of reagent into the reaction container 108 containing the sample. Thus, the sample and the reagent are mixed in the reaction container 108, and a measurement specimen is prepared.

The measurement specimen preparator 12 further comprises a reaction container transporter 175 for transferring the reaction container 108. The reaction container transporter 175 includes a movement mechanism in each of X, Y, and Z directions which are three orthogonal axes, and grips and transfers the reaction container 108. The reaction container transporter 175 transfers the reaction container 108 containing the measurement specimen from the holding hole 191 of the warming table 190 to a container placement section 131 of the detector 13. Further, the reaction container transporter 175 transfers the measured reaction container 108 from the container placement section 131 to a disposal port 106.

The detector 13 irradiates the measurement specimen in the reaction container 108 held in the container placement section 131 with light, senses transmitted light (light that has passed through the measurement specimen among light emitted from the light source 132) from the measurement specimen irradiated with light, and obtains optical information corresponding to an intensity of sensed light. The detector 13 includes: the container placement section 131 for arranging the reaction container 108 containing the measurement specimen; the light source 132 for irradiating the reaction container 108 with light for signal detection; and a light receiver 133 arranged to face the light source 132 with the reaction container 108 therebetween. The detector 13 comprises the container placement section 131 capable of holding a plurality of reaction containers 108, and obtains optical information in parallel for each measurement specimen in the plurality of reaction containers 108. That is, the detector 13 is configured to be capable of simultaneously obtaining optical information of a plurality of measurement specimens.

The detector 13 continuously obtains optical information of a process in which platelets in the measurement specimen in the reaction container 108 arranged in the container placement section 131 aggregate. The light source 132 irradiates the measurement specimen in the reaction container 108 arranged in the container placement section 131 with light continuously for a predetermined time. The light source 132 includes a light source such as a light emitting diode or a halogen lamp. The light receiver 133 continuously senses transmitted light from the measurement specimen in the reaction container 108 irradiated with light, and outputs an electric signal corresponding to an intensity of sensed light. The light receiver 133 includes a photoelectric conversion element that converts the sensed light into an electric signal and outputs the electric signal, and an A/D converter, digitizes this electric signal, and transmits digitalized signal to the control unit 3 via the controller 210. The detector 13 continuously senses the transmitted light from the measurement specimen from the start to the end of irradiation of the measurement specimen with light, outputs an electric signal corresponding to the intensity of sensed light, performs sampling at predetermined intervals, and performs digitization. Each obtained digital value is optical information indicating a transmitted light intensity at a corresponding timing. The measurement unit 2 transmits each digital value to the control unit 3 each time it is obtained. The sampling interval is not particularly limited, but is, for example, every 0.1 seconds, every 1 second, or the like.

The measurement unit 2 comprises a stirrer for stirring the measurement specimen. The measurement unit 2 senses the transmitted light from the measurement specimen irradiated with light by the detector 13 while stirring the measurement specimen in the reaction container 108 arranged in the container placement section 131 by the stirrer. In the present embodiment, a stirring bar is placed in the reaction container 108 used for the measurement of the platelet aggregation ability, and the stirrer rotates the stirring bar. The stirring bar is composed of a magnet. The stirrer is a so-called magnetic stirrer, is provided below the container placement section 131, and rotates the stirring bar by rotating a magnet. Note that a vibration-type stirrer can also be adopted as the stirrer. As will be described in detail later, platelet aggregation starts by stirring the first measurement specimen. Further, in a case where spontaneous platelet aggregation occurs in the second measurement specimen, spontaneous platelet aggregation starts by stirring the second measurement specimen.

The controller 213 of the control unit 3 analyzes the platelet aggregation ability of the sample and the like based on the optical information indicating the transmitted light intensity obtained by the detector 13.

FIG. 3 is a block diagram showing a configuration of the sample analyzer 1. As shown in FIG. 3, the measurement unit 2 includes a communication part 209 and a controller 210. Further, the control unit 3 includes a communication part 212 and a controller 213. The measurement unit 2 and the control unit 3 transmit and receive information via the communication parts 209 and 212. The controller 210 includes a processor such as a CPU (Central Processing Unit) and an FPGA (field-programmable gate array), and a storage device such as a ROM (Read Only Memory), a RAM (Random Access Memory), and a hard disk.

The processor of the controller 210 controls each component of the measurement unit 2 via an I/O board 211 by executing a control program stored in the storage device. The controller 210 controls, for example, dispensing operations of the dispensers 120a and 120b, dispensing operations of the dispensers 150a and 150b, operation of the reagent table 180, operations of each table and each reaction container transporter, operation of the detector 13, operation of the stirrer, and the like. The controller 210 controls the measurement specimen preparator 12, the detector 13, and the like so as to perform the first to third measurement operations and obtain the first to third optical information.

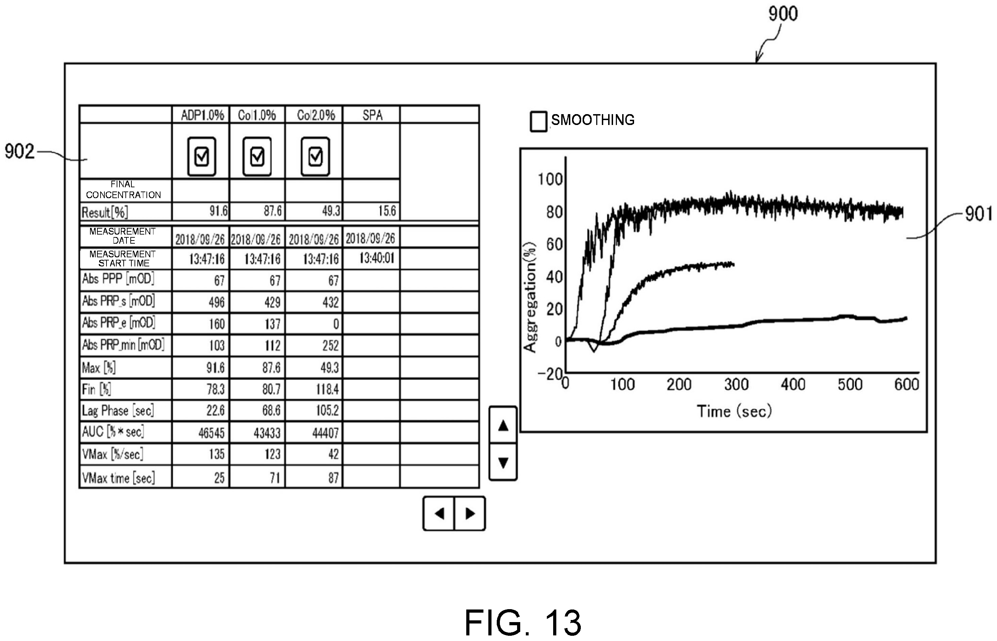

Similar to the controller 210 of the measurement unit 2, the controller 213 of the control unit 3 includes a processor such as a CPU, and a storage device such as a ROM, a RAM, and a hard disk. In the present embodiment, the controller 213 of the control unit 3 analyzes the platelet aggregation ability of the sample and the like based on the optical information obtained by the measurement unit 2. The display 4 is connected to the control unit 3, and analysis results by the control unit 3 such as an analysis result regarding the platelet aggregation ability as shown in FIG. 13 described later are displayed on the display 4.

Note that although the example shown in FIG. 3 shows a form including two controllers 210 and 213, the number of controllers is not particularly limited. One controller may control the measurement unit 2 and the control unit 3, or three or more controllers may control the measurement unit 2 and the control unit 3. Further, the arrangement of the controllers is not particularly limited. For example, the controller 210 of the measurement unit 2 may obtain an analysis result regarding the platelet aggregation ability of a blood sample based on the optical information obtained by the detector 13, or the controller 210 and the controller 213 may share the processing and obtain the analysis result regarding the platelet aggregation ability of the blood sample based on the optical information obtained by the detector 13.

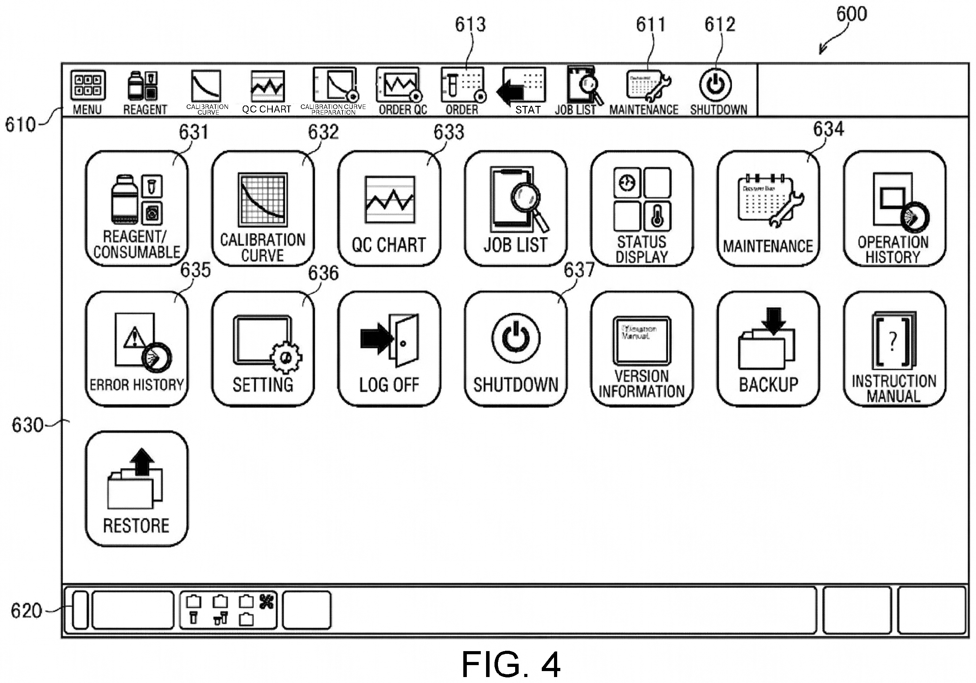

FIG. 4 shows a main menu screen 600, and FIG. 5 shows an order screen 700. The main menu screen 600 and the order screen 700 are displayed on the display 4.

As shown in FIG. 4, the main menu screen 600 includes a toolbar 610 including buttons for main functions, a status display area 620 including indicators indicating information such as a state of the apparatus, a state of a host computer, and remaining amounts of reagents, consumables, and the like, and a menu icon display area 630. The toolbar 610 includes a maintenance button 611 operated when performing maintenance and inspection of the apparatus, a shutdown button 612 operated when shutting down the apparatus, an order button 613 for displaying the order screen 700, and the like.

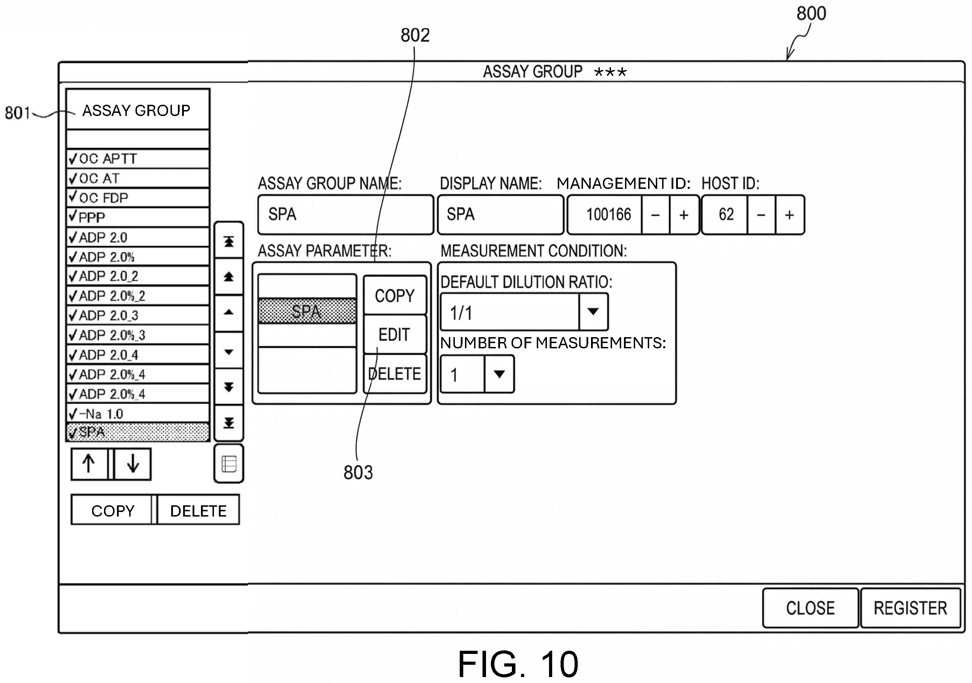

In the menu icon display area 630, a plurality of buttons (icons) for performing various operations and displaying information are displayed, and the same buttons as those included in the toolbar 610 may be displayed. The menu icon display area 630 includes a reagent/consumable button 631, a calibration curve button 632, a QC chart button 633, a maintenance button 634, an error history button 635, a setting button 636, a shutdown button 637, and the like. The setting button 636 is, for example, a button for displaying a setting screen regarding measurement of platelet aggregation ability and measurement of spontaneous platelet aggregation (see FIG. 10 described later).

As shown in FIG. 5, the order screen 700 includes an order registration section 701 for registering sample information such as a sample ID and measurement items, and a measurement type selection section 702 for displaying a measurement type being selected. In a case where the sample information is not registered in the host computer, the operator needs to input the sample information necessary for measurement into the order registration section 701. Further, the operator selects a measurement type by the measurement type selection section 702. The measurement type selection section 702 is provided with a pull-down type button, and this button functions as a measurement type selection button.

The order registration section 701 is displayed in a table format in which sample information can be input. In the measurement of platelet aggregation ability, a PRP sample and a PPP sample are arranged in the sample rack 105 as a pair, and the paired PRP sample and PPP sample are managed by the same sample ID. In the order registration section 701, a plurality of measurement items are displayed. The measurement items are, for example, types and concentrations of inducing agents. The operator can select desired measurement items for each sample from the table of the order registration section 701, and the selected measurement items are registered as an order.

Also in the case of performing measurement of spontaneous platelet aggregation, order registration is automatically performed based on information read by the sample information reader 103, or in a case where sample information is not registered in the host computer, order registration is performed based on information input to the order screen 700. In the table of the order registration section 701, a checkbox for SPA (spontaneous platelet aggregation) is displayed along with the measurement items. When manually registering measurement of spontaneous platelet aggregation, the operator checks the SPA checkbox. Thereby, the second measurement operation is performed by the measurement unit 2.

In the example shown in FIG. 5, the characters "platelet aggregation order" are displayed in the measurement type selection section 702, indicating that measurement of the platelet aggregation ability is selected as the measurement type. For example, in a state where the measurement of the platelet aggregation ability is selected as the measurement type, the SPA checkbox is operable. The measurement of the spontaneous platelet aggregation is optionally performed in association with the measurement of the platelet aggregation ability, but it may be possible to select a "spontaneous platelet aggregation order" in the measurement type selection section 702 to enable the measurement of the spontaneous platelet aggregation independent of the measurement of the platelet aggregation ability.

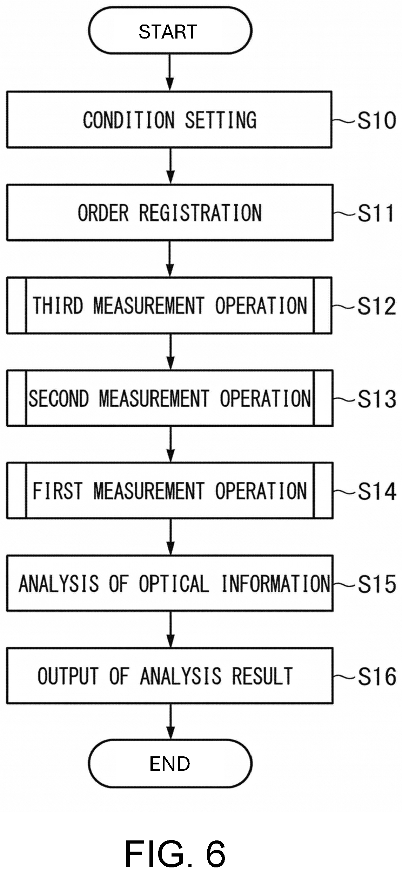

Hereinafter, a measurement method for obtaining an analysis result regarding the platelet aggregation ability will be described in detail with reference to FIGS. 6 to 9. The measurement for obtaining the analysis result regarding the platelet aggregation ability (hereinafter, simply referred to as "sample measurement") includes measurement of the platelet aggregation ability (normal aggregation) by the first measurement operation of the measurement unit 2, measurement of the spontaneous platelet aggregation by the second measurement operation, and measurement of the PPP sample by the third measurement operation.

As shown in FIG. 6, a sample analysis method which is an example of an embodiment includes steps of: setting measurement conditions (step S10); registering a measurement order (step S11); preparing measurement specimens and obtaining optical information (steps S12 to S14); and analyzing the optical information and outputting analysis results (steps S15 and S16). In the present embodiment, the controller 213 of the control unit 3 acquires a first measurement order regarding the first measurement operation, a second measurement order regarding the second measurement operation, and a third measurement order regarding the third measurement operation, and causes the measurement unit 2 to perform the first measurement operation, the second measurement operation, and the third measurement operation in accordance with the acquired measurement orders.

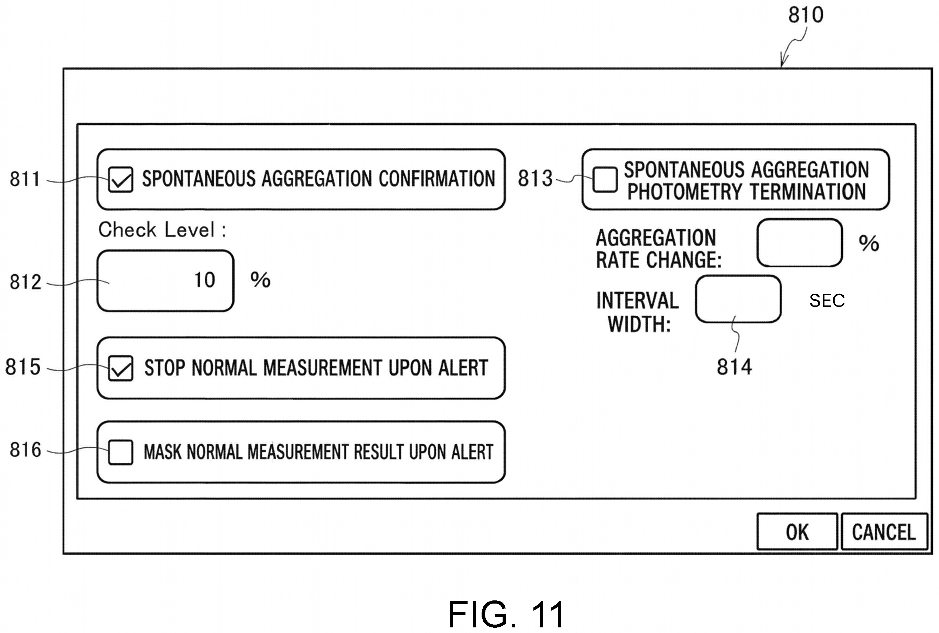

In step S10, the controller 213 of the control unit 3 sets measurement conditions based on, for example, information input to a predetermined setting screen (see FIG. 10 and FIG. 11 described later). As will be described in detail later, in the case of the second measurement operation, examples of the measurement conditions include a threshold for determining the presence or absence of spontaneous platelet aggregation, a measurement discontinuation condition, and the like.

In step S11, the controller 210 and the controller 213 control the sample information reader 103 to read the barcode of the sample container 104, inquire of the host computer for information necessary for measurement, and perform order registration. In a case where sample information is not registered in the host computer, the controller 213 performs order registration based on information input to the order screen 700. Note that in a case where the measurement of the spontaneous platelet aggregation is not registered in the host computer, or in a case where the SPA checkbox in the order screen 700 is not checked, the order registration for the second measurement operation is not performed, and the second measurement operation in step S13 is not performed.

In steps S12 to S14, the controller 210 performs the first to third measurement operations. In step S12, the controller 210 controls the measurement specimen preparator 12 to prepare a third measurement specimen containing a PPP sample, and controls the detector 13 to perform the third measurement operation for obtaining third optical information of the third measurement specimen. Next, in step S13, the controller 210 controls the measurement specimen preparator 12 to prepare a second measurement specimen containing a PRP sample and not containing an inducing reagent, and controls the detector 13 to perform the second measurement operation for obtaining second optical information of the second measurement specimen, that is, the measurement operation of spontaneous platelet aggregation.

In step S14, the controller 210 controls the measurement specimen preparator 12 to prepare a first measurement specimen containing a PRP sample and an inducing reagent, and controls the detector 13 to perform the first measurement operation for obtaining first optical information of the first measurement specimen, that is, the measurement operation of normal aggregation. As described above, in the present embodiment, digital values indicating transmitted light intensities are obtained as the first to third optical information. Note that the order of steps S12 to S14 is not particularly limited, and for example, they may be performed in the order of the first measurement operation, the second measurement operation, and the third measurement operation.

In step S15, the optical information obtained in steps S12 to S14 is transmitted to the control unit 3, and analysis of the optical information is performed by the controller 213 of the control unit 3. The controller 213 converts the transmitted light intensity received from the measurement unit 2 into absorbance indicating a degree to which light is absorbed by the measurement specimen. The transmitted light intensity increases as platelets aggregate, and the absorbance decreases as platelets aggregate. For example, the controller 213 calculates a temporal change in an aggregation rate of the first measurement specimen, assuming that an initial absorbance of the first measurement specimen immediately after start of stirring is an aggregation rate of 0% and an absorbance of the third measurement specimen is an aggregation rate of 100%, and creates a coagulation curve of normal aggregation (coagulation curve obtained by converting absorbance into aggregation rate) as shown in FIG. 13 described later. Further, a maximum aggregation rate of normal aggregation is calculated from a maximum change amount of absorbance accompanying a platelet aggregation reaction. The maximum aggregation rate of normal aggregation is an example of a measurement value of the platelet aggregation ability of a blood sample.

The maximum aggregation rate of normal aggregation can be calculated by, for example, the following formula (1): 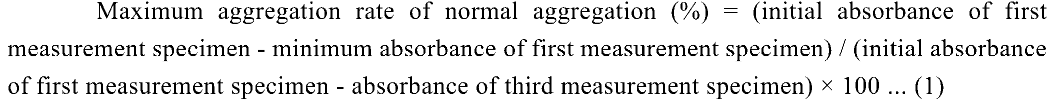 Maximum aggregation rate of normal aggregation (%) = (initial absorbance of first measurement specimen - minimum absorbance of first measurement specimen) / (initial absorbance of first measurement specimen - absorbance of third measurement specimen) × 100 (1)

The aggregation rate of normal aggregation at a predetermined time point can be calculated by, for example, the following formula (2): 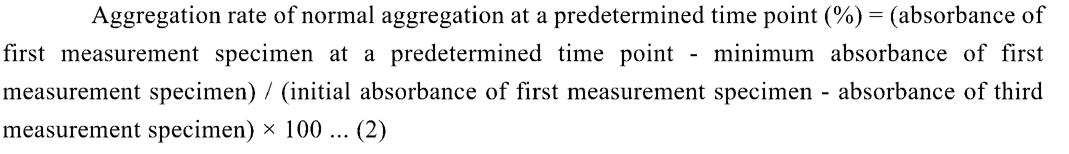 Aggregation rate of normal aggregation at a predetermined time point (%) = (absorbance of first measurement specimen at a predetermined time point - minimum absorbance of first measurement specimen) / (initial absorbance of first measurement specimen - absorbance of third measurement specimen) × 100 (2)

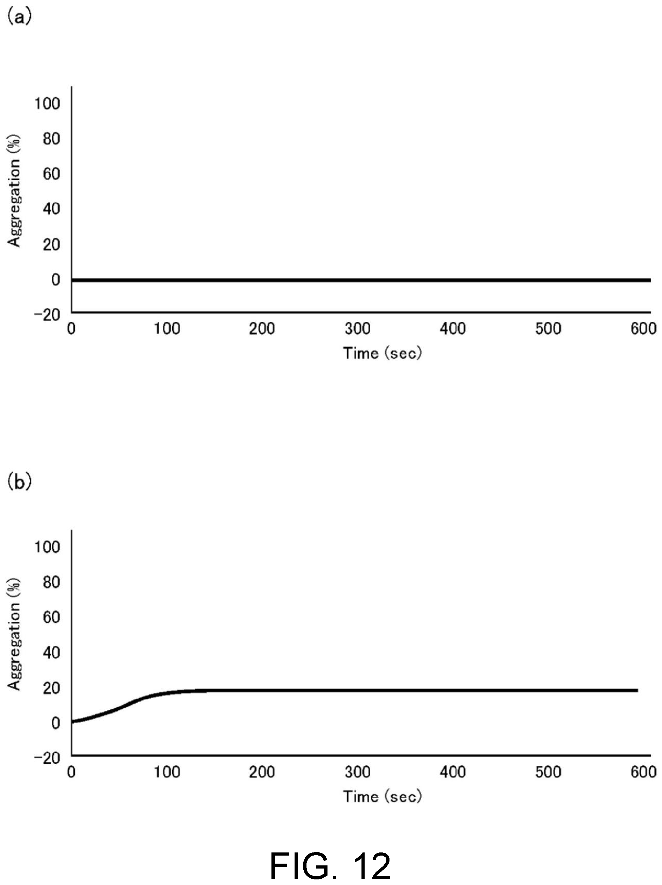

Further, for example, the controller 213 calculates a temporal change in an aggregation rate of the second measurement specimen, assuming that an initial absorbance of the second measurement specimen immediately after start of stirring is an aggregation rate of 0% and an absorbance of the third measurement specimen is an aggregation rate of 100%, and creates a coagulation curve of spontaneous aggregation as shown in (a) and (b) of FIG. 12 and FIG. 13 described later. Further, a maximum aggregation rate of spontaneous aggregation is calculated from a maximum change amount of absorbance accompanying a spontaneous platelet aggregation reaction. The coagulation curve of spontaneous aggregation and the maximum aggregation rate of spontaneous aggregation are examples of spontaneous aggregation information.

The maximum aggregation rate of spontaneous aggregation can be calculated by, for example, the following formula (3): 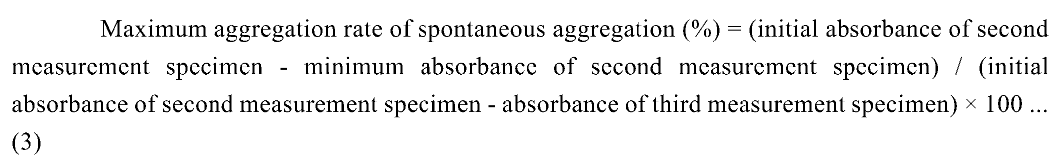 Maximum aggregation rate of spontaneous aggregation (%) = (initial absorbance of second measurement specimen - minimum absorbance of second measurement specimen) / (initial absorbance of second measurement specimen - absorbance of third measurement specimen) × 100 (3)

The aggregation rate of spontaneous aggregation at a predetermined time point can be calculated by, for example, the following formula (4): 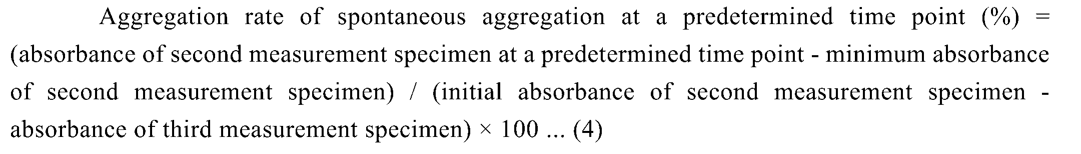 Aggregation rate of spontaneous aggregation at a predetermined time point (%) = (absorbance of second measurement specimen at a predetermined time point - minimum absorbance of second measurement specimen) / (initial absorbance of second measurement specimen - absorbance of third measurement specimen) × 100 (4)

In the present embodiment, the controller 213 of the control unit 3 executes conversion from transmitted light intensity to absorbance, creation of coagulation curves, and calculation of maximum aggregation rates, but at least one of these processes may be executed by the controller 210 of the measurement unit 2. Further, the conversion from transmitted light intensity to absorbance may be omitted, and the coagulation curve may be created from the transmitted light intensity.

In step S16, the controller 213 displays an analysis result of the platelet aggregation ability such as coagulation curves and maximum aggregation rates on the display 4. As will be described in detail later, spontaneous aggregation information may be displayed on a display screen of the analysis result together with a measurement value of the platelet aggregation ability (normal aggregation) as the analysis result.

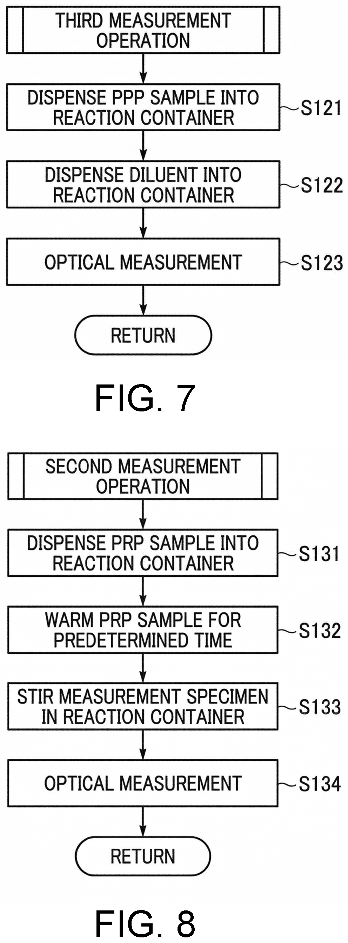

FIG. 7 is a flowchart showing an example of the third measurement operation in step S12 of FIG. 6. As shown in FIG. 7, in step S121, the controller 210 of the measurement unit 2 controls the dispenser 150a to aspirate a predetermined amount of the PPP sample from the sample container 104 containing the PPP sample and dispense it into the reaction container 108. In step S122, the controller 210 controls the dispenser 120a to aspirate a predetermined amount of diluent from the reagent container 50 containing the diluent and dispense the diluent into the reaction container 108 to prepare a third measurement specimen containing the PPP sample.

In step S123, the controller 210 controls the reaction container transporter 175 to transfer the reaction container 108 containing the third measurement specimen, which is the diluted PPP sample, to the detector 13, and controls the detector 13 to irradiate the third measurement specimen in the reaction container 108 with light from the light source 132 for a predetermined time to obtain a transmitted light intensity of the third measurement specimen as third optical information. The predetermined time can be arbitrarily set, and an example of the predetermined time is 300 seconds or more and 600 seconds or less. From the start to the end of the predetermined time, the detector 13 of the measurement unit 2 senses transmitted light from the third measurement specimen, outputs an electric signal corresponding to an intensity of the sensed light, performs sampling at predetermined intervals, and performs digitization. Each obtained digital value is third optical information indicating a transmitted light intensity at a corresponding timing. The measurement unit 2 transmits each digital value to the control unit 3 each time it is obtained. The sampling interval is not particularly limited, but is, for example, every 0.1 seconds, every 1 second, or the like. Note that step S122 may be omitted, and an undiluted PPP sample may be used as the third measurement specimen.

FIG. 8 is a flowchart showing an example of the second measurement operation in step S13 of FIG. 6. As shown in FIG. 8, in step S131, the controller 210 of the measurement unit 2 controls the dispenser 150a to aspirate a predetermined amount of the PRP sample from the sample container 104 containing the PRP sample, and dispense it into the reaction container 108 in which a stirring bar is placed, thereby preparing a second measurement specimen. The second measurement specimen in the present embodiment does not contain an inducing reagent, but may contain a predetermined amount of diluent. In step S132, the controller 210 controls the reaction container transporter 170 to transfer the reaction container 108 containing the second measurement specimen onto the warming table 190, and controls the warming table 190 to warm the second measurement specimen at a predetermined temperature for a predetermined time.

In step S133, the controller 210 controls the reaction container transporter 175 to transfer the reaction container 108 containing the second measurement specimen to the container placement section 131 of the detector 13, and controls the stirrer to rotate the stirring bar in the reaction container 108 to stir the second measurement specimen. In step S134, the controller 210 controls the detector 13 to irradiate the second measurement specimen in the reaction container 108 with light from the light source 132 for a predetermined time, and obtains a transmitted light intensity of the second measurement specimen as second optical information while stirring the second measurement specimen. That is, the controller 210 executes step S133 and step S134 in parallel. The predetermined time can be arbitrarily set, and an example of the predetermined time is 50 seconds or more and 600 seconds or less. The predetermined time (measurement time of the second measurement specimen) may be, for example, the same as the measurement time of the first measurement specimen, and may vary depending on success or failure of a discontinuation condition described later. From the start to the end of the predetermined time, the detector 13 of the measurement unit 2 senses transmitted light from the second measurement specimen, outputs an electric signal corresponding to an intensity of the sensed light, performs sampling at predetermined intervals, and performs digitization. Each obtained digital value is second optical information indicating a transmitted light intensity at a corresponding timing. The measurement unit 2 transmits each digital value to the control unit 3 each time it is obtained. The sampling interval is not particularly limited, but is, for example, every 0.1 seconds, every 1 second, or the like.

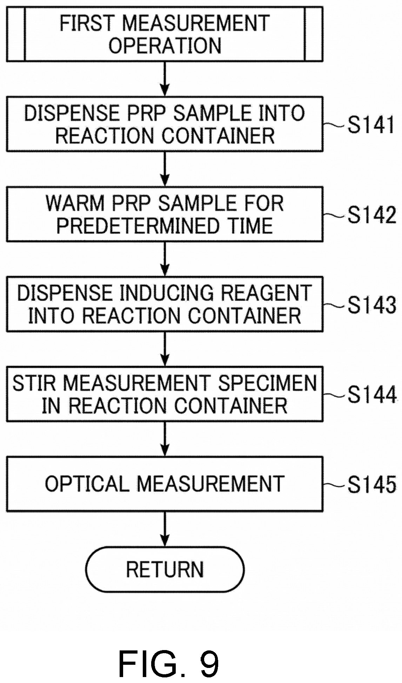

FIG. 9 is a flowchart showing an example of the first measurement operation in step S14 of FIG. 6. As shown in FIG. 9, in step S141, the controller 210 of the measurement unit 2 controls the dispenser 150a to aspirate a predetermined amount of the PRP sample from the sample container 104 containing the PRP sample, and dispense it into the reaction container 108 in which a stirring bar is placed. In step S142, the controller 210 controls the reaction container transporter 170 to transfer the reaction container 108 containing the PRP sample onto the warming table 190, and controls the warming table 190 to warm the PRP sample at a predetermined temperature for a predetermined time.

In step S143, the controller 210 controls the dispenser 120a to aspirate a predetermined amount of an inducing reagent from the reagent container 60 containing the inducing reagent, and dispense it into the reaction container 108 containing the PRP sample, thereby preparing a first measurement specimen containing the PRP sample and the inducing reagent. The controller 210 dispenses a predetermined amount of a predetermined inducing reagent based on measurement items registered as an order, and prepares one or more types of first measurement specimens for one PRP sample. In step S144, the controller 210 controls the reaction container transporter 175 to transfer the reaction container 108 containing the first measurement specimen to the container placement section 131 of the detector 13, and controls the stirrer to rotate the stirring bar in the reaction container 108 to stir the first measurement specimen.

In step S145, the controller 210 controls the detector 13 to irradiate the first measurement specimen in the reaction container 108 with light from the light source 132 for a predetermined time, and obtains a transmitted light intensity of the first measurement specimen as first optical information while stirring the first measurement specimen. That is, the controller 210 executes step S144 and step S145 in parallel. The predetermined time can be arbitrarily set, and an example of the predetermined time is 300 seconds or more and 600 seconds or less. From the start to the end of the predetermined time, the detector 13 of the measurement unit 2 senses transmitted light from the first measurement specimen, outputs an electric signal corresponding to an intensity of the sensed light, performs sampling at predetermined intervals, and performs digitization. Each obtained digital value is first optical information indicating a transmitted light intensity at a corresponding timing. The measurement unit 2 transmits each digital value to the control unit 3 each time it is obtained. The sampling interval is not particularly limited, but is, for example, every 0.1 seconds, every 1 second, or the like. As described above, the first measurement operation is similar to the second measurement operation except that the step S143 of dispensing an inducing reagent is added. In a case where a plurality of measurement items having different types and concentrations of inducing reagents are registered as an order for one PRP sample, steps S141 to S145 are repeated according to the number of measurement items. Preparation and measurement of first measurement specimens corresponding to a plurality of measurement items can be performed simultaneously and in parallel.

FIG. 10 is a view showing an example of a setting screen regarding sample measurement. As shown in FIG. 10, a setting screen 800 includes a list display area 801 in which a plurality of measurement items are displayed, and a parameter registration section 802 for confirming, setting, changing, or the like, conditions of each measurement item. As measurement items of the platelet aggregation ability (normal aggregation), for example, a plurality of items having different types and concentrations of inducing agents (inducing reagents) are registered. The setting screen 800 is a screen for confirming, setting, changing, or the like, conditions in each measurement item of the platelet aggregation ability of a sample, and it is also possible to newly register a measurement item or delete a registered measurement item on the setting screen 800.

The setting screen 800 is displayed on the display 4, for example, by operating the setting button 636 of the main menu screen 600. In the list display area 801, a plurality of measurement items are displayed side by side in a vertical direction of the screen. In the setting screen 800, by selecting a measurement item in the list display area 801, the selected measurement item is displayed in the parameter registration section 802. An edit button 803 is provided in the parameter registration section 802. An operator can set or change the conditions of the selected measurement item by operating the edit button 803.

The setting screen 800 is further used for setting or changing information necessary for measurement in the case of performing measurement of the spontaneous platelet aggregation. In the setting screen 800, "SPA", which is an item for spontaneous platelet aggregation, is displayed in the list display area 801 along with the measurement items of the platelet aggregation ability. When an operator selects SPA in the list display area 801, SPA is displayed in the parameter registration section 802, and measurement conditions and the like for the spontaneous platelet aggregation can be set or changed by operating the edit button 803.

FIG. 11 is a view showing an example of a setting screen regarding measurement of spontaneous platelet aggregation. As shown in FIG. 11, a setting screen 810 includes a selection button 811 for selecting whether or not to perform measurement of the spontaneous platelet aggregation of a PRP sample, and an input part 812 for inputting a threshold necessary for evaluation of the spontaneous platelet aggregation. The selection button 811 includes a checkbox. When an operator checks the checkbox for "spontaneous aggregation confirmation" of the selection button 811, the second measurement operation for performing measurement of the spontaneous platelet aggregation becomes effective. Further, in a case where the checkbox of the selection button 811 is checked, input of a threshold to the input part 812 may be enabled.

The setting screen 810 is displayed on the display 4, for example, by selecting SPA in the list display area 801 of the setting screen 800 shown in FIG. 10 and operating the edit button 803 of the parameter registration section 802. Alternatively, the setting screen 810 may be displayed on the display 4 by operating the setting button 636 of the main menu screen 600.

In a case where the checkbox of the selection button 811 is checked, a setting may be made such that the SPA checkbox in the order screen 700 shown in FIG. 5 is automatically checked. In this case, in principle, measurement of the spontaneous platelet aggregation of a sample is performed, and for example, if the check is removed on the order screen 700, the measurement of the spontaneous platelet aggregation is not performed only for the sample for which the check is removed. In a case where the checkbox of the selection button 811 is not checked, the measurement of the spontaneous platelet aggregation may be performed only for a checked sample by checking the SPA checkbox on the order screen 700.

A threshold input to the input part 812 serves as a criterion, for example, in determining presence or absence of spontaneous platelet aggregation. To the input part 812, a maximum aggregation rate of a second measurement specimen, at which it is generally determined that spontaneous platelet aggregation has occurred, is input as a threshold. The threshold of the maximum aggregation rate is not limited to a specific value, but as an example, is set to 5% or more and 25% or less. For example, in a case where the maximum aggregation rate of the second measurement specimen (for example, the maximum aggregation rate of spontaneous aggregation obtained by formula (3) above) is equal to or greater than the threshold, the controller 213 of the control unit 3 determines that there is spontaneous aggregation, and in a case where the maximum aggregation rate is less than the threshold, determines that there is no spontaneous aggregation.

The setting screen 810 further includes a selection button 813 for setting a condition for discontinuing measurement of the spontaneous platelet aggregation, and an input part 814 for inputting the condition for discontinuing measurement. The measurement of the spontaneous platelet aggregation is performed for a preset time, but if a change in aggregation rate of the second measurement specimen in a predetermined section shorter than the preset time is less than a predetermined value, it is assumed that the spontaneous platelet aggregation has substantially reached a saturated state and does not progress further, or the sample is one in which spontaneous platelet aggregation does not occur. For this reason, it is desirable to discontinue the measurement to achieve efficient operation of the sample analyzer 1. When a checkbox for "shorten spontaneous aggregation measurement" of the selection button 813 is checked, measurement of the spontaneous platelet aggregation is finished when a predetermined discontinuation condition is satisfied, even if the preset measurement time has not elapsed.

An example of the discontinuation condition input to the input part 814 is a condition regarding a change amount of aggregation rate in a predetermined section in which the second optical information of the second measurement specimen is obtained. To the input part 814 shown in FIG. 11, a section width (predetermined section) and a change amount of aggregation rate in the section width can be input. As a specific example, a case can be mentioned in which the change amount of aggregation rate is less than 2% in a section width of 50 seconds. In this case, the change amount of aggregation rate of the second measurement specimen is calculated every 50 seconds from start of measurement, and if the calculated change amount of aggregation rate is 2% or more, measurement is continued, and if it is less than 2%, measurement is discontinued. When the discontinuation condition is satisfied and the measurement is finished, the maximum aggregation rate at the time of measurement finish is output as a measurement result of spontaneous aggregation. Note that it is also possible to set, as the measurement discontinuation condition, that the maximum aggregation rate of the second measurement specimen has reached the threshold for determining the presence or absence of spontaneous platelet aggregation. In this case, the threshold input to the input part 812 can be used for determining success or failure of the discontinuation condition.

In the present embodiment, the second optical information obtained by the measurement unit 2 by preparing and measuring the second measurement specimen is transmitted from the measurement unit 2 to the control unit 3, and the controller 213 of the control unit 3 determines success or failure of the discontinuation condition. As described above, the measurement unit 2 continuously obtains the second optical information and transmits it to the control unit 3 each time it is obtained. After the measurement unit 2 starts obtaining the second optical information, during obtaining of the second optical information, if the second optical information obtained so far satisfies a predetermined discontinuation condition, the controller 213 causes the measurement unit 2 to discontinue measurement of the spontaneous platelet aggregation, that is, obtaining of the second optical information of the second measurement specimen. In a case where the discontinuation condition is a condition regarding a change amount of aggregation rate in a predetermined section width, the controller 213 determines that the discontinuation condition is satisfied if the change amount of aggregation rate based on the second optical information obtained in the section width is less than a predetermined threshold, and causes the measurement unit 2 to discontinue the measurement.

The setting screen 810 further includes a selection button 815 for selecting whether or not to discontinue measurement of the platelet aggregation ability (normal aggregation) when spontaneous platelet aggregation is detected, and a selection button 816 for selecting whether or not to mask measurement results of normal aggregation when spontaneous aggregation is detected. Each of the selection buttons 815 and 816 includes a checkbox. When a checkbox for "discontinue normal measurement upon alert" of the selection button 815 is checked, if spontaneous aggregation is detected, subsequent measurement of normal aggregation, that is, the first measurement operation is discontinued. When a checkbox for "mask normal measurement results upon alert" of the selection button 816 is checked, if spontaneous aggregation is detected, the measurement result of normal aggregation (maximum aggregation rate of normal aggregation) is masked. If neither checkbox of the selection buttons 815 and 816 is checked, when spontaneous aggregation is detected, an alert regarding reliability of the measurement result of normal aggregation is displayed along with the measurement result of normal aggregation (maximum aggregation rate of normal aggregation).

Since the platelet aggregation ability (normal aggregation) may not be correctly evaluated for a sample in which spontaneous platelet aggregation occurs, it may be desirable to discontinue the first measurement operation for the sample at a time point when spontaneous aggregation of the sample is detected. In this case, for example, consumption of expensive reagents can be suppressed, and efficient operation of the sample analyzer 1 can be achieved. Further, in a case where the first measurement operation is not discontinued, or in a case where the measurement operation could not be discontinued because the first measurement operation was in progress, it is preferable to mask the measurement result of normal aggregation of the sample for which spontaneous platelet aggregation has been confirmed, or display an alert together with the measurement result of normal aggregation, in terms of enhancing reliability of measurement of the sample analyzer 1. Note that the measurement unit 2 continues the first measurement operation in a case where spontaneous platelet aggregation is not detected. Further, in a case where the checkbox of the selection button 815 is not checked, the first measurement operation may be continued regardless of presence or absence of spontaneous aggregation.

The controller 213 of the control unit 3 may determine whether or not to control the measurement unit 2 to perform the first measurement operation based on spontaneous aggregation information regarding spontaneous platelet aggregation. This determination is executed when the checkbox of the selection button 815 is checked, but may be executed as a standard setting of the sample analyzer 1. In the present embodiment, the controller 213 causes the measurement unit 2 to discontinue the first measurement operation in a case where the maximum aggregation rate of the second measurement specimen reaches the threshold input to the input part 812. Upon acquiring the first measurement order regarding the first measurement operation, the controller 213 causes the measurement unit 2 to perform the first measurement operation, but discontinues the first measurement operation in a case where the maximum aggregation rate of the second measurement specimen reaches the threshold. In a case where the maximum aggregation rate of the second measurement specimen is less than the threshold, the first measurement operation is continued based on the first measurement order.

As described above, the controller 213 of the control unit 3 may cause the measurement unit 2 to discontinue the first measurement operation in a case where spontaneous aggregation information regarding spontaneous platelet aggregation satisfies a predetermined determination condition. An example of the predetermined determination condition is a condition regarding an aggregation rate of spontaneous aggregation. An example of the predetermined determination condition is whether or not the maximum aggregation rate of the second measurement specimen has reached the threshold input to the input part 812. In a case where information regarding spontaneous platelet aggregation satisfies the predetermined determination condition, i.e., in a case where the maximum aggregation rate of the second measurement specimen has reached the threshold, the controller 213 prohibits preparation of the first measurement specimen by the measurement unit 2. In a case where the first measurement specimen has already been prepared, the controller 213 may discontinue measurement of the first measurement specimen in the detector 13. Note that the first measurement operation to be discontinued is a first measurement operation for a first measurement specimen prepared using the same PRP sample as the second measurement specimen whose maximum aggregation rate has reached the threshold.

In a case where spontaneous aggregation information regarding spontaneous platelet aggregation satisfies a predetermined determination condition, the controller 213 of the control unit 3 may output, to the display 4, a screen for accepting whether or not to perform the first measurement operation. In this case, when the maximum aggregation rate of the second measurement specimen reaches the threshold, the controller 213 outputs, to the display 4, an acceptance screen for accepting whether or not to perform the first measurement operation, and allows an operator to determine whether or not to discontinue the first measurement operation. This acceptance screen includes selection buttons for selecting discontinuation or continuation of the first measurement operation. Based on the operator's operation of the selection button on the acceptance screen, the controller 213 discontinues the first measurement operation in a case where an operation signal regarding measurement discontinuation is acquired, and continues the first measurement operation in a case where an operation signal regarding measurement continuation is acquired.

The controller 213 of the control unit 3 determines whether or not to display a measurement value of the platelet aggregation ability based on spontaneous aggregation information regarding spontaneous platelet aggregation. This determination is executed when the checkbox of the selection button 816 is checked, but may be executed as a standard setting of the sample analyzer 1. In a case where the spontaneous aggregation information regarding spontaneous platelet aggregation satisfies a predetermined determination condition, the controller 213 may display, on the display 4, an analysis result not including a measurement value of the platelet aggregation ability, or an analysis result including the measurement value of the platelet aggregation ability and an alert. An example of the predetermined determination condition is a condition regarding an aggregation rate of spontaneous aggregation. An example of the predetermined determination condition is whether or not the maximum aggregation rate of the second measurement specimen has reached the threshold input to the input part 812. Note that the measurement value that is not displayed is a measurement value of a first measurement specimen prepared using the same PRP sample as the second measurement specimen whose maximum aggregation rate has reached the threshold.

For example, in a case where the maximum aggregation rate of the second measurement specimen reaches the threshold, the controller 213 of the control unit 3 does not display the measurement value of the platelet aggregation ability (normal aggregation) on the display 4, and displays other information regarding platelet aggregation such as the maximum aggregation rate of the second measurement specimen. In this case, since there is a possibility that the platelet aggregation ability is not correctly measured, reliability of the sample analyzer 1 is improved by not displaying the measurement value. Further, in a case where the maximum aggregation rate of the second measurement specimen reaches the threshold, the controller 213 may display the measurement value of the platelet aggregation ability along with an alert regarding reliability of the measurement value of the platelet aggregation ability. Examples of the alert include reminder information such as: spontaneous platelet aggregation of the sample has been detected; there is a possibility that the platelet aggregation ability is not correctly evaluated due to the spontaneous platelet aggregation; and the PRP sample is not appropriate as a sample for measuring the platelet aggregation ability.

(a) and (b) of FIG. 12 shows an example of measurement results (coagulation curves) of spontaneous platelet aggregation. (a) of FIG. 12 shows measurement results in a case where spontaneous aggregation does not occur, and (b) of FIG. 12 shows measurement results in a case where spontaneous aggregation occurs.

As shown in (a) of FIG. 12, in a case where the horizontal axis represents elapsed time from start of measurement (start of obtaining optical information) and the vertical axis represents an aggregation rate determined based on the second optical information of the second measurement specimen, and a temporal change in the aggregation rate is displayed, in measurement results of a sample in which spontaneous platelet aggregation does not occur, the aggregation rate is 0%. In this case, it is determined that there is no spontaneous aggregation, and processes such as discontinuation of the first measurement operation, non-display of measurement values of the platelet aggregation ability, and display of an alert are not executed. On the other hand, as shown in (b) of FIG. 12, in measurement results of a sample in which spontaneous platelet aggregation occurs, the aggregation rate increases from early start of measurement and reaches about 20%. Since a threshold for determining presence or absence of spontaneous aggregation is generally set to less than 20%, in this case, it is determined that there is spontaneous aggregation, and processes such as discontinuation of the first measurement operation, non-display of measurement values of the platelet aggregation ability, and/or display of an alert are executed.

In the measurement results shown in (a) of FIG. 12, the aggregation rate does not change from 0% even after the measurement time has elapsed. Further, regarding the measurement results shown in (b) of FIG. 12, after the measurement time has elapsed 130 seconds, the aggregation rate does not change and is substantially constant. To continue measurement in such a case is not preferable from a viewpoint of efficient operation of the sample analyzer 1. For this reason, in a case where information regarding spontaneous platelet aggregation satisfies a predetermined discontinuation condition, the controller 213 preferably causes the measurement unit 2 to discontinue measurement of the spontaneous platelet aggregation. In the present embodiment, as described above, a change amount of aggregation rate of the second measurement specimen in a predetermined section is set as a measurement discontinuation condition, and if the change amount of aggregation rate is less than a predetermined threshold, it is determined that the discontinuation condition is satisfied, and measurement of the spontaneous platelet aggregation is discontinued. For example, in a case where the section width is 50 seconds, the change amount of aggregation rate in a section from 150 seconds to 200 seconds is almost 0%, which is less than a predetermined threshold (e.g., 2%), so measurement of the spontaneous platelet aggregation is discontinued at 200 seconds from start of measurement.

FIG. 13 shows a result display screen 900 which is an example of a result display screen including an analysis result regarding the platelet aggregation ability. Note that FIG. 13 shows results in a case where spontaneous platelet aggregation has occurred.

As shown in FIG. 13, the result display screen 900 includes a first display area 901 that displays coagulation curves, and a second display area 902 that displays a result list including information such as measurement date and time, absorbance, and maximum aggregation rate. The coagulation curve is a curve obtained by converting absorbance of a measurement specimen into an aggregation rate as described above. The first display area 901 and the second display area 902 include measurement results of a plurality of measurement items, i.e., measurement results of a plurality of first measurement specimens, and according to the result display screen 900, comparison of measurement results is easy. The result display screen 900 is a screen that simultaneously displays measurement results of a plurality of measurement items, but the result display screen may be, for example, a screen that includes measurement item tabs and individually displays detailed results of each measurement item by selecting a tab. Further, a plurality of types of result display screens may exist, and each result display screen may be switchable.

On the result display screen 900, as analysis results regarding the platelet aggregation ability, measurement results of spontaneous platelet aggregation (spontaneous aggregation information) are displayed along with measurement results of the platelet aggregation ability (normal aggregation). In the second display area 902, a column displayed as "SPA" is provided, and the maximum aggregation rate of spontaneous aggregation is displayed in this column. The maximum aggregation rate of spontaneous aggregation can be calculated using, for example, the formula (3) above. The first display area 901 displays a graph in which the horizontal axis represents elapsed time from start of measurement and the vertical axis represents aggregation rate, and displays a coagulation curve of spontaneous platelet aggregation along with coagulation curves of normal aggregation. In this example, the coagulation curve with the smallest aggregation rate is the coagulation curve of spontaneous aggregation, and in a case of a sample in which spontaneous aggregation does not occur, the aggregation rate does not change from 0%. Note that each coagulation curve may be displayed such that line types are different from each other, and an identification display may be attached to each coagulation curve. Note that the spontaneous aggregation information does not need to be displayed on the same screen as the measurement result of the platelet aggregation ability (normal aggregation), and may be displayed on different screens.

In the example shown in FIG. 13, since the maximum aggregation rate of spontaneous aggregation exceeds 15%, the sample is generally determined to have spontaneous platelet aggregation. In a case where it is determined that there is spontaneous aggregation, only the maximum aggregation rate of spontaneous aggregation may be displayed on the result display screen 900, and measurement results of the platelet aggregation ability may be masked. A method for masking measurement results is not particularly limited, and as an example, a method of displaying as "**%" can be mentioned.

The result display screen 900 is displayed on the display 4 according to an instruction from the controller 213. When the measurement unit 2 performs the first measurement operation, the controller 213 obtains a measurement value regarding the platelet aggregation ability of a sample based on first optical information, and displays, on the display 4, the result display screen 900 including an analysis result regarding the platelet aggregation ability based on the measurement value regarding the platelet aggregation ability. When the measurement unit 2 performs the second measurement operation, the controller 213 obtains spontaneous aggregation information regarding spontaneous platelet aggregation of a sample based on second optical information, and displays, on the display 4, the result display screen 900 including an analysis result regarding the platelet aggregation ability based on the spontaneous aggregation information. When the measurement unit 2 performs the first measurement operation and the second measurement operation, the controller 213 obtains a measurement value regarding the platelet aggregation ability of a sample based on first optical information and obtains spontaneous aggregation information regarding spontaneous platelet aggregation of the sample based on second optical information, and displays, on the display 4, the result display screen 900 including an analysis result regarding the platelet aggregation ability based on the measurement value regarding the platelet aggregation ability and the spontaneous aggregation information. When the measurement unit 2 performs the first measurement operation and does not perform the second measurement operation, the controller 213 obtains a measurement value regarding the platelet aggregation ability of a sample based on first optical information, and displays, on the display 4, the result display screen 900 including an analysis result regarding the platelet aggregation ability based on the measurement value regarding the platelet aggregation ability. When the measurement unit 2 performs the first measurement operation, the second measurement operation, and the third measurement operation, the controller 213 obtains a measurement value regarding the platelet aggregation ability of a sample based on first and third optical information and obtains spontaneous aggregation information regarding spontaneous platelet aggregation of the sample based on second and third optical information, and displays, on the display 4, the result display screen 900 including an analysis result regarding the platelet aggregation ability based on the measurement value regarding the platelet aggregation ability and the spontaneous aggregation information.

The spontaneous aggregation information regarding spontaneous platelet aggregation displayed on the display 4 preferably includes at least one of: information indicating whether or not the sample is appropriate as a PRP sample for measuring the platelet aggregation ability (hereinafter, referred to as "sample suitability information"); a coagulation curve of spontaneous platelet aggregation; and a maximum aggregation rate of spontaneous platelet aggregation. The analysis result regarding the platelet aggregation ability displayed on the display 4 preferably includes a graph displaying coagulation curves of normal aggregation and a coagulation curve of spontaneous aggregation. The coagulation curve of normal aggregation is a curve showing a temporal change of the first optical information of the first measurement specimen, and the coagulation curve of spontaneous aggregation is a curve showing a temporal change of the second optical information of the second measurement specimen. The result display screen 900 includes information other than the sample suitability information among these information. An example of the sample suitability information includes an alert indicating that the PRP sample is not appropriate as a sample for measuring the platelet aggregation ability.

The controller 213 displays the alert on the display 4 as the sample suitability information in a case where the maximum aggregation rate of the second measurement specimen reaches the threshold. Further, in a case where the maximum aggregation rate of the second measurement specimen is less than the threshold, information indicating that the PRP sample is appropriate as a sample for measuring the platelet aggregation ability may be displayed on the display 4 as the sample suitability information.

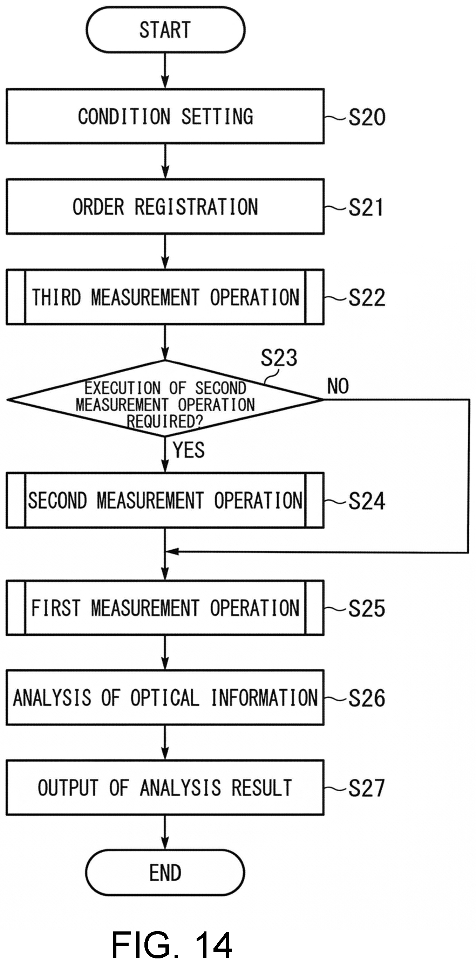

Hereinafter, another example of the sample analysis method will be described with reference to FIGS. 14 to 17. In the following, differences from the above embodiment will be mainly described, and redundant descriptions for contents common to the above embodiment will be omitted.

The sample analysis method shown in FIG. 14 is the same as the method shown in FIG. 6 except that a step S23 of determining whether or not to perform the second measurement operation is added. In a case where the controller 213 of the control unit 3 acquires a second measurement order regarding the second measurement operation, the controller 213 causes the measurement unit 2 to perform the second measurement operation (Yes in step S23, S24). On the other hand, in a case where the controller 213 has not acquired the second measurement order, the controller 213 skips the second measurement operation in step S24 and causes the measurement unit 2 to perform the first measurement operation (No in step S23, S25). Note that the order of the third measurement operation in step S22, the second measurement operation in step S24, and the first measurement operation in step S25 is not particularly limited.

In the method shown in FIG. 14, the second measurement operation, which is the measurement of spontaneous platelet aggregation, is optionally performed in association with the first measurement operation, which is the measurement of the platelet aggregation ability. That is, for the PRP sample transported to the measurement unit 2, processing is not performed such that the second measurement operation is performed while the first measurement operation is not performed. The first measurement operation in step S25 is also performed based on the measurement order registered in step S21, but differs from the case of the first measurement operation in that step S23 is a step of determining whether or not to perform an optional measurement.

In step S23, the controller 213 may cause the measurement unit 2 to perform the second measurement operation in a case where the checkbox for "spontaneous aggregation confirmation" of the selection button 811 shown in FIG. 11 is checked. If the checkbox of the selection button 811 is checked, the second measurement operation may be performed regardless of presence or absence of a second measurement order. Alternatively, the controller 213 may be configured to register a second measurement order in a case where the checkbox of the selection button 811 is checked.

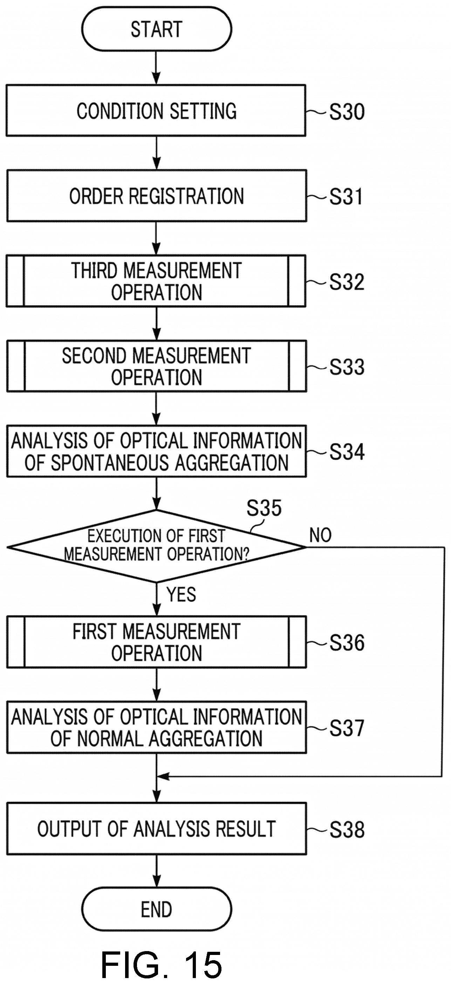

The sample analysis method shown in FIG. 15 differs from the method shown in FIG. 6 in which the first measurement operation is performed regardless of the measurement result of spontaneous platelet aggregation, in that whether or not to perform the first measurement operation is determined (steps S34, S35) based on the measurement result of spontaneous platelet aggregation obtained by the second measurement operation in step S33. In steps S33 and S34, the controller 213 causes the measurement unit 2 to perform the second measurement operation, analyzes the obtained second optical information, and determines, for example, presence or absence of spontaneous platelet aggregation. As described above, the controller 213 determines that there is spontaneous aggregation in a case where the maximum aggregation rate of the second measurement specimen reaches the threshold input to the input part 812 shown in FIG. 11, and determines that there is no spontaneous aggregation in a case where the maximum aggregation rate is less than the threshold.

In steps S35 to S37, in a case where the maximum aggregation rate of the second measurement specimen is less than the threshold, i.e., in a case where it is determined that there is no spontaneous platelet aggregation, the controller 213 causes the measurement unit 2 to perform the first measurement operation and analyzes the obtained first optical information. On the other hand, in a case where the maximum aggregation rate of the second measurement specimen reaches the threshold, i.e., in a case where it is determined that there is spontaneous platelet aggregation, the controller 213 skips the first measurement operation in steps S36 and S37 and displays an analysis result on the display 4 (step S38). In this case, the measurement result of spontaneous platelet aggregation is displayed on the display 4. As described above, since the platelet aggregation ability may not be correctly evaluated for a sample in which spontaneous platelet aggregation occurs, consumption of reagents can be suppressed and efficient operation of the sample analyzer 1 can be achieved by not performing the first measurement operation for the sample for which spontaneous aggregation has been confirmed.

In the method shown in FIG. 15, it is determined whether or not to perform the first measurement operation after completion of the measurement of spontaneous platelet aggregation, but if the frequency of occurrence of spontaneous aggregation is low, the first measurement operation may be started before completion of the measurement of spontaneous platelet aggregation, and whether or not to discontinue the first measurement operation may be determined based on the measurement result of spontaneous platelet aggregation. In a case where the maximum aggregation rate of the second measurement specimen reaches the threshold, the controller 213 prohibits preparation of the first measurement specimen by the measurement unit 2. Note that if the first measurement specimen has already been prepared at the time point when it is determined that there is spontaneous platelet aggregation, measurement of optical information of the first measurement specimen is discontinued. In this case, consumption of reagents can be suppressed while realizing rapid sample measurement.

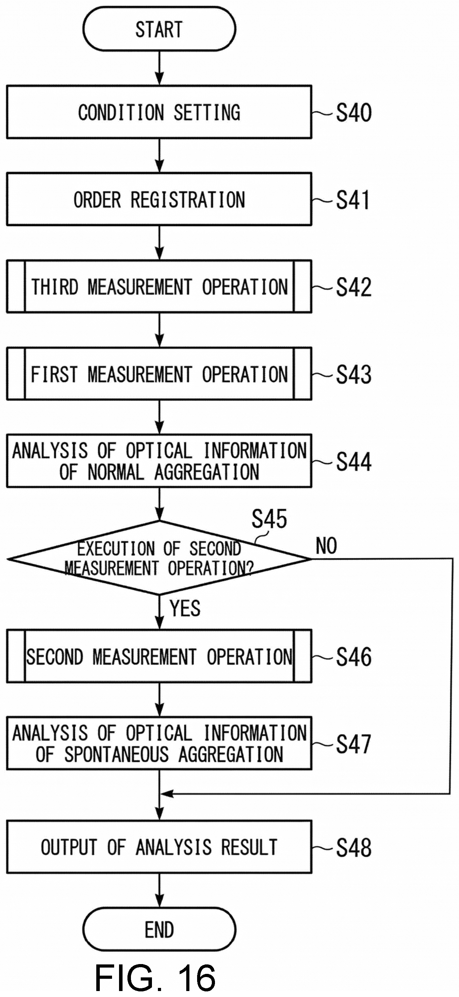

The sample analysis method shown in FIG. 16 is common to the method shown in FIG. 15 in that it is determined whether or not to perform a next measurement operation based on a measurement result regarding platelet aggregation. On the other hand, the method shown in FIG. 16 differs from the method shown in FIG. 15 in that whether or not to control the measurement unit 2 to perform the second measurement operation is determined based on the measurement value of the platelet aggregation ability obtained by the first measurement operation. In the method shown in FIG. 16, for example, in a case where an abnormality is recognized in the measurement result of the platelet aggregation ability, measurement of spontaneous platelet aggregation is performed for the purpose of verifying the cause of the abnormality. The controller 213 may determine that there is an abnormality in the measurement result of the platelet aggregation ability, for example, in a case where the maximum aggregation rate of the first measurement specimen is equal to or greater than a predetermined threshold, or in a case where the coagulation curve of the first measurement specimen has an abnormal shape.

In steps S43 and S44, the controller 213 causes the measurement unit 2 to perform the first measurement operation, analyzes the obtained first optical information, and obtains measurement results regarding the platelet aggregation ability such as coagulation curves and maximum aggregation rates. In step S45, the controller 213 determines whether or not to control the measurement unit 2 to perform the second measurement operation based on the measurement result regarding the platelet aggregation ability. For example, in a case where the maximum aggregation rate of the first measurement specimen is equal to or greater than a predetermined threshold, the controller 213 causes the second measurement operation in step S46 to be performed. In a case where the maximum aggregation rate of the first measurement specimen is less than the predetermined threshold, the second measurement operation in steps S46 and S47 is skipped, and an analysis result is displayed on the display 4 (step S48). In this case, the measurement result of the platelet aggregation ability is displayed on the display 4.

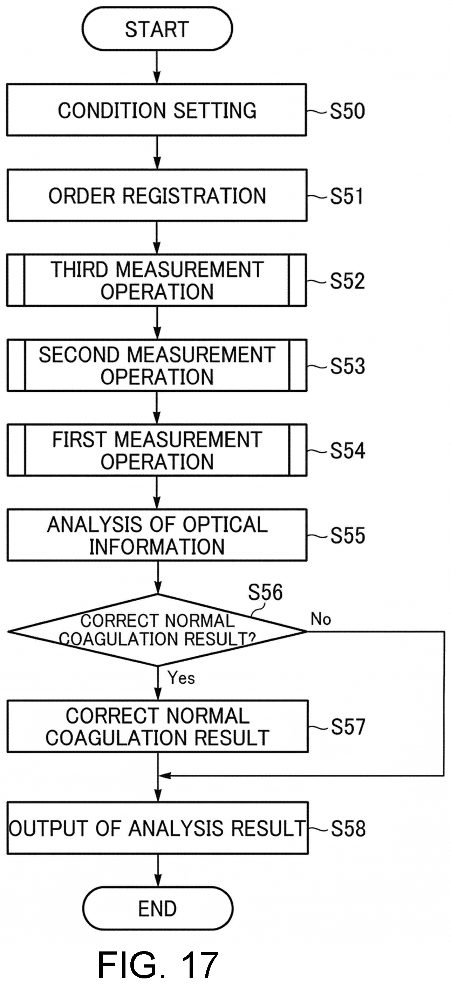

The sample analysis method shown in FIG. 17 differs from the method shown in FIG. 6 in that steps S56 and S57 of determining whether or not to correct information regarding the platelet aggregation ability (normal aggregation) obtained by the first measurement operation and performing the correction in a case where a predetermined condition is satisfied are added. In steps S56 and S57, in a case where the spontaneous aggregation information regarding spontaneous platelet aggregation satisfies a predetermined determination condition, the controller 213 corrects the information regarding the platelet aggregation ability based on the spontaneous aggregation information. In a case where the information regarding spontaneous platelet aggregation does not satisfy the predetermined determination condition, the correction process in step S57 is skipped, and uncorrected measurement results are displayed on the display 4 (step S58).

An example of the predetermined determination condition in step S56 is whether or not the maximum aggregation rate of the second measurement specimen has reached the threshold input to the input part 812 shown in FIG. 11. In step S56, the controller 213 determines that correction of the measurement result of the platelet aggregation ability (measurement result of normal aggregation) is necessary in a case where the maximum aggregation rate of the second measurement specimen has reached the threshold. In a case where the maximum aggregation rate of the second measurement specimen is less than the threshold, it is determined that correction of the measurement result of normal aggregation is unnecessary (No in step S56), and the correction process in step S57 is skipped.

In step S57, the controller 213 corrects the maximum aggregation rate of normal aggregation based on, for example, the maximum aggregation rate of the second measurement specimen, i.e., the maximum aggregation rate of spontaneous platelet aggregation. In this case, in step S58, corrected measurement results are displayed on the display 4. A correction method of the measurement result of normal aggregation is not particularly limited, but examples of the correction method include subtracting the maximum aggregation rate of spontaneous aggregation from the maximum aggregation rate of normal aggregation, and multiplying the maximum aggregation rate of normal aggregation by a predetermined coefficient determined according to the maximum aggregation rate of spontaneous aggregation. The predetermined coefficient is a value less than 1, and is predetermined, for example, by an experiment or the like.

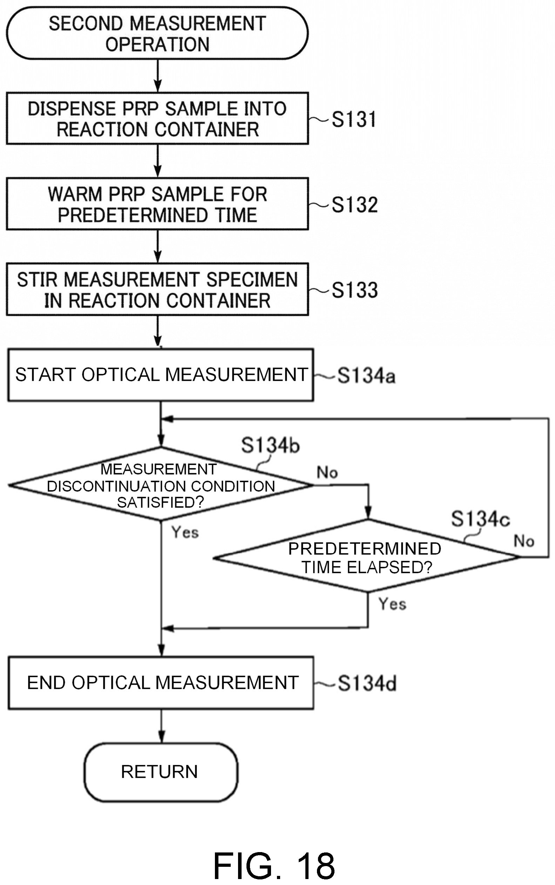

FIG. 18 is a flowchart showing another example of the second measurement operation. The second measurement operation shown in FIG. 18 differs from the second measurement operation shown in FIG. 8 in that, in step S134a, after start of obtaining the transmitted light intensity (second optical information) of the second measurement specimen prepared in step S131 and warmed in step S132 while stirring the specimen, success or failure of a measurement discontinuation condition is determined. During obtaining of the second optical information, in a case where the information obtained so far satisfies a predetermined discontinuation condition (Yes in step S134b), the controller 213 causes the measurement unit 2 to discontinue measurement of transmitted light, i.e., obtaining of the second optical information (step S134d).

Step S134b is executed in a case where the checkbox for "shorten spontaneous aggregation measurement" of the selection button 813 shown in FIG. 11 is checked, but may be executed as a standard setting of the sample analyzer 1. The discontinuation condition is, for example, a condition regarding a change amount of aggregation rate in a predetermined section in which optical information of the second measurement specimen is obtained. In a case where the change amount of the aggregation rate of the second measurement specimen calculated from the second optical information is less than a predetermined threshold, the controller 213 determines that the discontinuation condition is satisfied (Yes in step S134b) and causes the measurement unit 2 to discontinue obtaining of the second optical information (step S134d).

On the other hand, during obtaining of the second optical information, in a case where the information obtained so far does not satisfy the predetermined discontinuation condition (No in step S134b), the controller 213 continues measurement of absorbance until a predetermined time has elapsed, and finishes measurement of absorbance when the predetermined time has elapsed (Yes in step S134c, S134d). Specifically, if the change amount of the aggregation rate of the second measurement specimen calculated from the second optical information is equal to or greater than a predetermined threshold, measurement of transmitted light is continued to obtain the second optical information. Although not shown in FIG. 8, passage of time in step S134c is also determined in the second measurement operation shown in FIG. 8.

In the above embodiment, the PPP sample is measured together with the PRP sample, but measurement of the PPP sample may be omitted. In this case, as the absorbance of the third measurement specimen in the formulas (1) to (4) above, a predetermined value acquired in advance by an experiment or the like may be used.

As described above, according to the sample analyzer 1 and the sample analysis method having the above configurations, information regarding spontaneous platelet aggregation of a blood sample can be easily known. Thereby, in a case where the degree of spontaneous platelet aggregation is large, reagents and time can be saved by avoiding measurement of the platelet aggregation ability for the first measurement specimen to which a reagent is added in advance. Further, reliability in testing of the platelet aggregation ability can be enhanced by displaying an alert in the measurement result of the platelet aggregation ability, not displaying the measurement result, or correcting the measurement result according to presence or absence and a degree of spontaneous aggregation.
The present disclosure includes following items 1-27.

Item 1. A sample analyzer configured to analyze a platelet aggregation ability of a blood sample, comprising:
a measurement unit comprising:
a measurement specimen preparator configured to prepare a measurement specimen in a reaction container, the measurement specimen preparator comprising:
(a1) a sample dispenser configured to dispense a plasma sample prepared from the blood sample into the reaction container, and
(a2) a reagent dispenser configured to dispense a reagent into the reaction container; and a detector configured to optically interrogate the measurement specimen, the detector comprising:
   (b1) a light source configured to irradiate the measurement specimen in the reaction container with light, and
   (b2) a light receiver configured to sense transmitted light or scattered light from the measurement specimen irradiated with light, wherein the detector is configured to obtain optical information corresponding to an intensity of the sensed transmitted or scattered light,
   wherein the measurement unit is capable of performing:
      a first measurement operation of preparing the measurement specimen in a first reaction container by dispensing a platelet-rich plasma sample as the plasma sample and an inducing reagent for inducing platelet aggregation as the reagent, and optically interrogating the measurement specimen in which the platelet aggregation is induced in the presence of the inducing reagent, and
      a second measurement operation of preparing a reference specimen in a second reaction container by dispensing the platelet-rich plasma sample without the inducing reagent, and optically interrogating the reference specimen in which a spontaneous platelet aggregation can be observed in the absence of the induced platelet aggregation; and
      a controller configured to control the measurement unit,
      wherein the controller is configured to execute a first analysis of the platelet aggregation ability of the blood sample based on the optical information obtained by the first measurement operation, and the controller is further configured to execute a second analysis of a spontaneous platelet aggregation of the blood sample based on the optical information obtained by the second measurement operation.

Item 2. The sample analyzer according to item 1, wherein
the measurement unit further comprises a stirrer configured to stir the measurement specimen in the reaction container, and
the controller is further configured to control the light source, the light receiver, and the stirrer to sense, by the light receiver, the transmitted or the scattered light from the measurement specimen irradiated with light while stirring the measurement specimen in the reaction container by the stirrer.

Item 3. The sample analyzer according to item 2, wherein
the reaction container contains a stirring bar, and
the stirrer is configured to rotate the stirring bar in the reaction container to stir the measurement specimen in the reaction container.

Item 4. The sample analyzer according to item 1, wherein
the controller is further configured to output an analysis result including spontaneous aggregation information obtained by the second analysis.

Item 5. The sample analyzer according to item 1, wherein
the controller is further configured to output an analysis result based on a measurement value of the platelet aggregation ability obtained by the first analysis and spontaneous aggregation information obtained by the second analysis.

Item 6. The sample analyzer according to item 1, wherein
the controller is further configured to output an analysis result including a measurement value of the platelet aggregation ability obtained by the first analysis.

Item 7. The sample analyzer according to item 1, wherein
the measurement unit is further capable of performing:
a third measurement operation of preparing a second reference specimen in a third reaction container by dispensing a platelet-poor plasma sample as the plasma sample, and optically interrogating the second reference specimen in which platelets are hardly included.

Item 8. The sample analyzer according to item 7, wherein
the controller is configured to execute the first analysis based on the optical information obtained by the first measurement operation and the optical information obtained by the third measurement operation, and
the controller is configured to execute the second analysis based on the optical information obtained by the second measurement operation and the optical information obtained by the third measurement operation.

Item 9. The sample analyzer according to item 1, wherein
the controller is further configured to acquire a measurement order and control the measurement unit to perform at least one of the first measurement operation and the second measurement operation in accordance with the acquired measurement order.

Item 10. The sample analyzer according to item 1, wherein
the controller is further configured to determine whether to control the measurement unit to perform the first measurement operation for the same blood sample based on spontaneous aggregation information obtained by the second analysis.

Item 11. The sample analyzer according to item 1, wherein
the controller is further configured to determine whether spontaneous aggregation information obtained by the second analysis satisfies a predetermined determination condition, and the controller is further configured to, in response to the spontaneous aggregation information satisfying the determination condition, output a screen for accepting whether or not to perform the first measurement operation for the same blood sample.

Item 12. The sample analyzer according to item 1, wherein
the controller is further configured to determine whether spontaneous aggregation information obtained by the second analysis satisfies a predetermined determination condition, and the controller is further configured to, in response to the spontaneous aggregation information satisfying the determination condition, prohibit performing of the first measurement operation for the same blood sample.

Item 13. The sample analyzer according to item 1, wherein
the controller is further configured to determine whether spontaneous aggregation information obtained by the second analysis satisfies a predetermined determination condition, and the controller is further configured to, in response to the spontaneous aggregation information satisfying the determination condition, output an analysis result including a measurement value of the platelet aggregation ability obtained by the first analysis and an alert regarding reliability of the measurement value.

Item 14. The sample analyzer according to item 1, wherein
the controller is further configured to determine whether spontaneous aggregation information obtained by the second analysis satisfies a predetermined determination condition, and the controller is further configured to, in response to the spontaneous aggregation information satisfying the determination condition, output an analysis result not including a measurement value of the platelet aggregation ability obtained by the first analysis.

Item 15. The sample analyzer according to item 1, wherein
the controller is further configured to determine whether spontaneous aggregation information obtained by the second analysis satisfies a predetermined determination condition, and the controller is further configured to, in response to the spontaneous aggregation information satisfying the determination condition, correct, based on the spontaneous aggregation information, a measurement value of the platelet aggregation ability obtained by the first analysis.

Item 16. The sample analyzer according to item 1, wherein
the controller is further configured to, upon start of performing the second measurement operation by the measurement unit, determine whether spontaneous aggregation information obtained so far by the second analysis satisfies a predetermined discontinuation condition, and
the controller is further configured to, in response to the spontaneous aggregation information obtained so far satisfying the discontinuation condition, control the measurement unit to discontinue obtaining of the optical information by the second measurement operation.

Item 17. The sample analyzer according to item 16, wherein
the discontinuation condition is a condition regarding a rate of change of the intensities of the sensed transmitted or scattered light.

Item 18. The sample analyzer according to item 1, wherein
the controller is further configured to determine whether to control the measurement unit to perform the second measurement operation for the same blood sample based on a measurement value of the platelet aggregation ability obtained by the first analysis.

Item 19. The sample analyzer according to item 1, wherein
spontaneous aggregation information obtained by the second analysis includes at least one of information indicating whether or not the platelet-rich plasma sample is appropriate for measuring the platelet aggregation ability; a coagulation curve of spontaneous platelet aggregation; and a maximum aggregation rate of spontaneous platelet aggregation.

Item 20. The sample analyzer according to item 1, wherein
the controller is further configured to output an analysis result including a first graph displaying a first curve indicating a temporal change of the intensities of the sensed transmitted or scattered light by the first measurement operation and a second curve indicating a temporal change of the intensities of the sensed transmitted or scattered light by the second measurement operation.

Item 21. The sample analyzer according to item 1, further comprising a transport unit configured to transport a sample container containing the plasma sample to a sample aspiration position of the measurement unit where the plasma sample is aspirated by the sample dispenser.

Item 22. The sample analyzer according to item 1, wherein
The measurement unit further comprises a reaction container transporter configured to transport the reaction container to the detector.

Item 23. The sample analyzer according to item 22, wherein
the measurement specimen preparator further comprises a warmer, wherein the reaction container transporter is configured to transport the reaction container from the warmer to the detector, and
the warmer is configured to warm the measurement specimen contained in the reaction container to a predetermined temperature.

Item 24. The sample analyzer according to item 1, wherein
the detector comprises a plurality of light sources and a plurality of light receivers corresponding to each of the light sources, and
the detector is configured to simultaneously interrogate a plurality of measurement specimens in a plurality of reaction containers.

Item 25. A sample analysis method for analyzing a platelet aggregation ability of a blood sample, comprising:
preparing, by a measurement specimen preparator of a measurement unit, a reference specimen in a reaction container, the reference specimen containing a platelet-rich plasma sample prepared from the blood sample and not containing an inducing reagent for inducing platelet aggregation, wherein the preparing the reference specimen includes dispensing the platelet-rich plasma sample without the inducing reagent into the reaction container;
optically interrogating, by a detector of the measurement unit, the reference specimen by irradiating the reference specimen in the reaction container with light and sensing transmitted light or scattered light from the reference specimen irradiated with light, whereby obtaining optical information corresponding to an intensity of the sensed transmitted or scattered light from the reference specimen; and
executing an analysis of a spontaneous platelet aggregation of the blood sample based on the optical information obtained by optically interrogating the reference specimen.

Item 26. The sample analysis method according to item 25, further comprising:
preparing, by the measurement specimen preparator, a measurement specimen in another reaction container, the measurement specimen containing the platelet-rich plasma sample and the inducing reagent, wherein the preparing the measurement specimen includes dispensing the platelet-rich plasma sample and the inducing reagent into said another reaction container;
optically interrogating, by the detector, the measurement specimen by irradiating the measurement specimen in said another reaction container with light and sensing transmitted light or scattered light from the measurement specimen irradiated with light, whereby obtaining optical information corresponding to an intensity of the sensed transmitted or scattered light from the measurement specimen;
executing an analysis of the platelet aggregation ability of the blood sample based on the optical information obtained by optically interrogating the measurement specimen.

Item 27. The sample analysis method according to item 26, further comprising:
outputting an analysis result including at least one of spontaneous aggregation information obtained by the analysis of the spontaneous platelet aggregation and a measurement value of the platelet aggregation ability obtained by the analysis of the platelet aggregation ability.

## Claims

1. A sample analyzer configured to analyze a platelet aggregation ability of a blood sample, comprising:
a measurement unit comprising:
a measurement specimen preparator configured to prepare a measurement specimen in a reaction container, the measurement specimen preparator comprising:
(a1) a sample dispenser configured to dispense a plasma sample prepared from the blood sample into the reaction container, and
(a2) a reagent dispenser configured to dispense a reagent into the reaction container; and
a detector configured to optically interrogate the measurement specimen, the detector comprising:
(b1) a light source configured to irradiate the measurement specimen in the reaction container with light, and
(b2) a light receiver configured to sense transmitted light or scattered light from the measurement specimen irradiated with light, wherein the detector is configured to obtain optical information corresponding to an intensity of the sensed transmitted or scattered light,
wherein the measurement unit is capable of performing:
a first measurement operation of preparing the measurement specimen in a first reaction container by dispensing a platelet-rich plasma sample as the plasma sample and an inducing reagent for inducing platelet aggregation as the reagent, and optically interrogating the measurement specimen in which the platelet aggregation is induced in the presence of the inducing reagent, and
a second measurement operation of preparing a reference specimen in a second reaction container by dispensing the platelet-rich plasma sample without the inducing reagent, and optically interrogating the reference specimen in which a spontaneous platelet aggregation can be observed in the absence of the induced platelet aggregation; and
a controller configured to control the measurement unit,
wherein the controller is configured to execute a first analysis of the platelet aggregation ability of the blood sample based on the optical information obtained by the first measurement operation, and the controller is further configured to execute a second analysis of a spontaneous platelet aggregation of the blood sample based on the optical information obtained by the second measurement operation.

2. The sample analyzer according to claim 1, wherein
the measurement unit further comprises a stirrer configured to stir the measurement specimen in the reaction container, and
the controller is further configured to control the light source, the light receiver, and the stirrer to sense, by the light receiver, the transmitted or the scattered light from the measurement specimen irradiated with light while stirring the measurement specimen in the reaction container by the stirrer.

3. The sample analyzer according to claim 2, wherein
the reaction container contains a stirring bar, and
the stirrer is configured to rotate the stirring bar in the reaction container to stir the measurement specimen in the reaction container.

4. The sample analyzer according to any one of claims 1 to 3, wherein
the controller is further configured to output an analysis result including spontaneous aggregation information obtained by the second analysis.

5. The sample analyzer according to any one of claims 1 to 4, wherein
the controller is further configured to output an analysis result based on a measurement value of the platelet aggregation ability obtained by the first analysis and spontaneous aggregation information obtained by the second analysis.

6. The sample analyzer according to any one of claims 1 to 5, wherein
the controller is further configured to output an analysis result including a measurement value of the platelet aggregation ability obtained by the first analysis.

7. The sample analyzer according to any one of claims 1 to 6, wherein
the measurement unit is further capable of performing:
a third measurement operation of preparing a second reference specimen in a third reaction container by dispensing a platelet-poor plasma sample as the plasma sample, and optically interrogating the second reference specimen in which platelets are hardly included.

8. The sample analyzer according to claim 7, wherein
the controller is configured to execute the first analysis based on the optical information obtained by the first measurement operation and the optical information obtained by the third measurement operation, and
the controller is configured to execute the second analysis based on the optical information obtained by the second measurement operation and the optical information obtained by the third measurement operation.

9. The sample analyzer according to any one of claims 1 to 8, wherein
the controller is further configured to acquire a measurement order and control the measurement unit to perform at least one of the first measurement operation and the second measurement operation in accordance with the acquired measurement order.

10. The sample analyzer according to any one of claims 1 to 9, wherein
the controller is further configured to determine whether spontaneous aggregation information obtained by the second analysis satisfies a predetermined determination condition, and
the controller is further configured to, in response to the spontaneous aggregation information satisfying the determination condition, output an analysis result including a measurement value of the platelet aggregation ability obtained by the first analysis and an alert regarding reliability of the measurement value.

11. The sample analyzer according to any one of claims 1 to 10, wherein
spontaneous aggregation information obtained by the second analysis includes at least one of information indicating whether or not the platelet-rich plasma sample is appropriate for measuring the platelet aggregation ability; a coagulation curve of spontaneous platelet aggregation; and a maximum aggregation rate of spontaneous platelet aggregation.

12. The sample analyzer according to any one of claims 1 to 11, wherein
the controller is further configured to output an analysis result including a first graph displaying a first curve indicating a temporal change of the intensities of the sensed transmitted or scattered light by the first measurement operation and a second graph displaying a second curve indicating a temporal change of the intensities of the sensed transmitted or scattered light by the second measurement operation.

13. The sample analyzer according to any one of claims 1 to 12, wherein
The measurement unit further comprises a reaction container transporter configured to transport the reaction container to the detector.

14. The sample analyzer according to any one of claims 1 to 13, wherein
the detector comprises a plurality of light sources and a plurality of light receivers corresponding to each of the light sources, and
the detector is configured to simultaneously interrogate a plurality of measurement specimens in a plurality of reaction containers.

15. A sample analysis method for analyzing a platelet aggregation ability of a blood sample, comprising:
preparing, by a measurement specimen preparator of a measurement unit, a reference specimen in a reaction container, the reference specimen containing a platelet-rich plasma sample prepared from the blood sample and not containing an inducing reagent for inducing platelet aggregation, wherein the preparing the reference specimen includes dispensing the platelet-rich plasma sample without the inducing reagent into the reaction container;
optically interrogating, by a detector of the measurement unit, the reference specimen by irradiating the reference specimen in the reaction container with light and sensing transmitted light or scattered light from the reference specimen irradiated with light, whereby obtaining optical information corresponding to an intensity of the sensed transmitted or scattered light from the reference specimen;
executing an analysis of a spontaneous platelet aggregation of the blood sample based on the optical information obtained by optically interrogating the reference specimen;
preparing, by the measurement specimen preparator, a measurement specimen in another reaction container, the measurement specimen containing the platelet-rich plasma sample and the inducing reagent, wherein the preparing the measurement specimen includes dispensing the platelet-rich plasma sample and the inducing reagent into said another reaction container;
optically interrogating, by the detector, the measurement specimen by irradiating the measurement specimen in said another reaction container with light and sensing transmitted light or scattered light from the measurement specimen irradiated with light, whereby obtaining optical information corresponding to an intensity of the sensed transmitted or scattered light from the measurement specimen; and
executing an analysis of the platelet aggregation ability of the blood sample based on the optical information obtained by optically interrogating the measurement specimen.
